# EUROPÄISCHE PATENTSCHRIFT

(11) **EP 1 161 524 B1**
(45) Veröffentlichungstag und Bekanntmachung des Hinweises auf die Patenterteilung: **21.02.2007**
(21) Anmeldenummer: 00910788.9
(22) Anmeldetag: 08.03.2000
(51) Int. Cl.: C12N 9/64, C07K 14/47, A61P 25/28

(54) **ZELLEN, DIE EIN AMYLOIDVORLAUFERPROTEIN UND EIN A-SEKRETASE COEXPRIMIEREN UND DEREN ANWENDUNGEN IN TESTVERFAHREN UND DIAGNOSTIK**
CELLS COEXPRESSING AN AMYLOID PRECURSOR PROTEIN AND AN $g(a)-SECRETASE AND THEIR USES IN TEST METHODS AND DIAGNOSTICS
CELLULES COEXPRIMANT UNE PROTEINE PRECURSEUR AMYLOIDE ET UNE $g(a)-SECRETASE, ET LEURS UTILISATIONS DANS DES METHODES D'ESSAI ET A DES FINS DE DIAGNOSTIC

(30) Priorität: 08.03.1999 DE 19910108
(43) Veröffentlichungstag der Anmeldung: 12.12.2001
(73) Patentinhaber: Fahrenholz, Falk, 55130 Mainz (DE)
(72) Erfinder: FAHRENHOLZ, Falk, D-55130 Mainz (DE); POSTINA, Rolf, D-64380 Rossdorf (DE)
(74) Vertreter: Isenbruck, Günter
(86) Internationale Anmeldenummer: PCT/EP2000/002018
(87) Internationale Veröffentlichungsnummer: WO 2000/056871

(56) Entgegenhaltungen:
- WO-A-98/30709
- WO-A-99/51752
- DE-A- 19 849 073
- SKOVRONSKY DANIEL M ET AL: "Protein kinase C-dependent alpha-secretase competes with beta-secretase for cleavage of amyloid-beta precursor protein in the trans-Golgi network." JOURNAL OF BIOLOGICAL CHEMISTRY, Bd. 275, Nr. 4, 28. Januar 2000 (2000-01-28), Seiten 2568-2575, XP000926127 ISSN: 0021-9258
- KOMANO HIROTO ET AL: "Involvement of cell surface glycosyl-phosphatidylinositol-linked aspartyl proteases in alpha-secretase-type cleavage and ectodomain solubilization of human Alzheimer beta-amyloid precursor protein in yeast." JOURNAL OF BIOLOGICAL CHEMISTRY, Bd. 273, Nr. 48, 27. November 1998 (1998-11-27), Seiten 31648-31651, XP000926126 ISSN: 0021-9258
- HINES VICTORIA ET AL: "The expression and processing of human beta-amyloid peptide precursors in Saccharomyces cerevisiae: Evidence for a novel endopeptidase in the yeast secretory system." CELLULAR & MOLECULAR BIOLOGY RESEARCH, Bd. 40, Nr. 4, 1994, Seiten 273-284, XP000952040
- MILLICHIP MARK I ET AL: "The metallo-disintegrin ADAM10 (MADM) from bovine kidney has type IV collagenase activity in vitro." BIOCHEMICAL AND BIOPHYSICAL RESEARCH COMMUNICATIONS, Bd. 245, Nr. 2, 17. April 1998 (1998-04-17), Seiten 594-598, XP000946859 ISSN: 0006-291X
- KÄRKKÄINEN IIVARI ET AL: "Metalloprotease-disintegrin (ADAM) genes are widely and differentially expressed in the adult CNS." MOLECULAR AND CELLULAR NEUROSCIENCE, Bd. 15, Nr. 6, Juni 2000 (2000-06), Seiten 547-560, XP000952183 ISSN: 1044-7431
- MICANOVIC R ET AL: "SELECTIVITY AT THE CLEAVAGE-ATTACHMENT SITE OF PHOSPHATIDYLINOSITOL-GLYCAN ANCHORED MEMBRANE PROTEINS IS ENZYMATICALLY DETERMINED" PROCEEDINGS OF THE NATIONAL ACADEMY OF SCIENCES OF THE UNITED STATES, Bd. 87, Nr. 20, 1990, Seiten 157-161, XP002155834 1990 ISSN: 0027-8424
- LAMMICH SVEN ET AL: "Constitutive and regulated alpha-secretase cleavage of Alzheimer's amyloid precursor protein by a disintegrin metalloprotease." PROCEEDINGS OF THE NATIONAL ACADEMY OF SCIENCES OF THE UNITED STATES, Bd. 96, Nr. 7, 30. März 1999 (1999-03-30), Seiten 3922-3927, XP002155835 March 30, 1999 ISSN: 0027-8424

## Beschreibung

Die Erfindung betrifft zunächst Zellen, die rekombinierte Nukleinsäure(n) enthalten, die ein Amyloidvorläuferprotein (APP) und eine α- Sekretase codieren, oder zumindest Teile der genannten Proteine, ferner ein Testverfahren zur Identifikation α-Sekretase-aktiver Substanzen sowie eines zur Identifikation weiterer Sekretasen, schließlich auch ein Verfahren zur Bestimmung der Anfälligkeit gegenüber neurodegenerativen Erkrankungen, insbesondere gegenüber *Morbus Alzheimer* sowie die Verwendung von Nukleinsäuren, die für eine α-Sekretase codieren, für die Gentherapie.

*Morbus Alzheimer* ist die häufigste Ursache für Demenz in der westlichen Welt und die vierthäufigste Todesursache in den USA.
Die *Alzheimer-*Inzidenz steigt mit dem Alter: 1 bis 5 % der Menschen über 65 und 10 bis 20 % über 80 sind betroffen.
*Alzheimer* ist neuropathologisch durch die Anwesenheit einer Vielzahl von neuritischen Plaques und neurofibrilären Fehlstrukturen gekennzeichnet, die zu einem massiven Verlust an Neuronen führt. Weder die Plaques noch die neurofibrilären Fehlstrukturen sind spezifisch für *Alzheimer* - sie kommen bei älteren, intellektuell unauffälligen Personen und bei gewissen anderen Krankheiten ebenfalls vor. Allerdings sind diese Strukturen bei *Alzheimer* zahlreicher und weiter verbreitet als bei nicht betroffenen Individuen. Während Plaques extrazellulär auftreten, findet man die neurofibrilären Fehlstrukturen in degenerierenden Neuronen. Der Hauptbestandteil der Plaques ist ein 39-43 Aminosäure (4kDa) großes Peptid, genannt β-Amyloid oder einfach β-Peptid (Aβ). Ein Mikrotubuli-assoziiertes Protein, Tau, ist der Hauptbestandteil der neurofibrilären Fehlstruk-turen (Überblick: Encyclopedia of Molecular Biology and Molecular Medicine, Band 1, Seiten 42 bis 52, Herausgeber Meyers, Robert A., VCH Verlag Weinheim)

Das β-Peptid (Aβ) stammt von dem Amyloidvorläuferprotein (amyloid precursor protein, APP) ab, einem Typ 1 Transmembranprotein, das in vielen Säugerzelltypen exprimiert wird.

Die Aβ-Sequenz umfaßt 28 Aminosäuren des extrazellulären und 12 bis 15 Aminosäurereste des membrandurchspannenden Teils von APP. Aβ wird aus APP mittels proteolytischer Prozessierung durch eine bisher nicht identifizierte Protease gebildet, der β-Sekretase, die am Aminoterminus schneidet und durch die am C-Terminus schneidende γ-Sekretase.

Der Hauptabbauweg von APP ist der konstitutive sekretorische Weg, der die Spaltung des Proteins innerhalb der Aß-Sequenz durch eine putative α-Sekretase an der Zelloberfläche (Sisodia, S.S., (1992) Proc. Natl. Acad. Sci. USA 89, Seiten 6075 bis 6079; Haass et al. (1992) Nature 357, Seiten 500 bis 503; Ikezu et al. (1998) J. Biol. Chem. 273, Seiten 10485 bis 10495) und im Trans-Golgi-System (Kuentzel et al. (1993) Biochem. J. 295, Seiten 367 bis 378; De Strooper et al. (1993) J. Cell Biol. 121, Seiten 295 bis 304; Sambamurti et al. (1992) J. Neurosci. Res. 33, Seiten 319 bis 329; Haass, et al. (1995) J. Cell. Biol. 128, Seiten 537 bis 547; Tomita et al. (1998) J. Biol. Chem. 273, Seiten 6277 bis 6284) umfaßt. Lösliche aminoterminale APP-Fragmente von 105 bis 125 kDa (sogenannte APPsα-Fragmente; Weidemann et al. (1989) Cell 57, Seiten 115 bis 126; Evin et al. (1994) Amyloid: Int. J. Exp. Clin. Invest. 1, Seiten 263 bis 280) werden ins extrazelluläre Medium freigesetzt. Das membrangebundene 10kDa große carboxyterminale Fragment (p10), das bei der α-Sekretase-Spaltung ebenfalls entsteht, enthält nur einen Teil des amyloidogenischen Aβ-Peptids und kann durch die γ-Sekretase zu einem 3kDa-Fragment (p3) weiter abgebaut werden (Haass et al. (1993) J. Biol. Chem. 268, Seiten 3021 bis 3024). Da der Abbau von APP durch eine α-Sekretase zu keinem intakten Aß-Peptid führt, ist er nicht amyloidogenisch, d.h. er führt nicht zur Bildung von Amyloidplaques und somit nicht zu *Morbus Alzheimer.*

Aus diesem Grund steht der α-Sekretase-Abbau von APP seit Jahren im Fokus des Forscherinteresses. Durch Boseman et al. (1994) (J. Biol. Chem. 269, Seiten 3111 bis 3116) wurde ein Fusionsprotein aus dem aminotermialen Teil von APP und einer alkalischen Phosphatase in H4-Zellen heterolog exprimiert, um die endogene α-Sekretaseaktivität dieser Zellen zu untersuchen und gegebenenfalls das entsprechende Protein zu identifizieren. Hierbei wurde der unter verschiedenen Bedingungen freigesetzte Anteil an Proteolyseprodukt mit Antikörpern bestimmt, die gegen alkalische Phosphatase gerichtet waren. Diese Versuche führten bisher nicht zur Identifikation der α-Sekretase. Vermutlich ist die Menge an endogener α-Sekretase zu gering, als daß eine direkte Isolierung und Identifikation der α-Sekretase möglich wäre.

Es ist Aufgabe der Erfindung, Zellen bereitzustellen, die in der Lage sind, ein Amyloidvorläuferprotein und eine α-Sekretase in ungewöhnlich großer Menge zu exprimieren. Eine weitere Aufgabe ist es, Zellen bereitzustellen, deren endogene α-Sekretaseaktivität vermindert ist. Es ist ferner Aufgabe der Erfindung, ein Verfahren zur Auffindung pharmazeutischer Wirksubstanzen anzugeben, die in den α-Sekretasestoffwechsel eingreifen, sowie ein Verfahren zur Identifizierung weiterer Sekretasen. Schließlich ist Aufgabe der Erfindung, ein weiteres Verfahren zur Bestimmung der Anfälligkeit gegenüber neurodegenerativen Erkrankungen, insbesondere gegenüber *Morbus Alzheimer* anzugeben sowie ein Arzneimittel gegen neurodegenartive Erkrankungen, insbesondere gegen *Morbus Alzheimer* bereitzustellen. Ferner sollen Tiermodelle wie transgene nicht-menschliche Tiere zum Studium von neurodegenerativen Erkrankungen, insbesondere von *Morbus Alzheimer* bereitgestellt werden.

Die Erfindung besteht in einer rekombinanten Zelle, die ein Substratprotein exprimiert, das mindestens einen Sequenzbereich von 18 Aminosäuren des humanen Amyloidvorläuferproteins (APP, amyloid precursor protein) umfasst oder das mindestens einen Sequenzbereich von 18 Aminosäuren eines Proteins umfaßt, welches zum humanen APP eine Sequenzidentität von 86% über mindestens 770 Aminosäuren besitzt, wobei der Sequenzbereich die α-Sekretasespaltstelle sowie 6 Aminosäurereste aminoterminal und 12 Aminosäurereste carboxyterminal bezogen auf die α-Sekretasespaltstelle enthält, und
a) entweder eine rekombinante Nukleinsäure enthält, umfassend mindestens ein Gen für X die Protease ADAM 10 aus Rind (Bos taurus) oder Mensch oder ein Gen für ein Proteaseprotein, das mit der Disintegrin-Metalloprotease ADAM 10 aus dem Rind (Bos taurus) oder aus dem Menschen eine Sequenzidentität von mindestens 94 % über 824 Aminosäuren auf Aminosäureebene aufweist, wobei das Gen oder die Gene unter der Kontrolle eines heterologen Promotors stehen,
b) oder heterologe RNA codierend für das besagte Proteaseprotein nach Alternative a) enthält.

Eine weitere Ausführungsform betrifft eine rekombinante Zelle, dadurch gekennzeichnet, daß sie rekombinante Nukleinsäure enthält, umfassend entweder
a) mindestens ein Gen für ein Substratprotein, das mindestens einen Sequenzbereich von 18 Aminosäuren des humanen Amyloidvorläuferproteins (APP, amyloid precursor protein) oder eines homologen Proteins umfasst oder das mindestens einen Sequenzbereich von 18 Aminosäuren eines Proteins umfaßt, welches zum humanen APP eine Sequenzidentität von 86% über mindestens 770 Aminosäuren besitzt, wobei der Sequenzbereich die α-Sekretasespaltstelle sowie 6 Aminosäurereste aminoterminal und 12 Aminosäurereste carboxyterminal bezogen auf die α-Sekretasespaltstelle enthält, und
b) mindestens ein Gen für die Protease ADAM 10 aus Rind (Bos taurus) oder Mensch oder ein Gen für ein Proteaseprotein, das mit der Disintegrin-Metalloprotease ADAM 10 aus dem Rind (Bos taurus) oder aus dem Menschen eine Sequenzidentität von mindestens 94 % über 824 Aminosäuren auf Aminosäureebene aufweist, wobei das Gen oder die Gene unter der Kontrolle eines heterologen Promotors stehen,
   oder dadurch gekennzeichnet, daß die Zelle heterologe RNA kodierend für das besagte Substratprotein nach a) und das besagte Proteaseprotein nach b) enthält.

Es wurde überraschenderweise gefunden, daß die an sich schon bekannte Disintegrin-Metalloprotease ADAM 10 die Eigenschaften der authentischen α-Sekretase aufweist. Die Disintegrin-Metalloprotease ADAM 10 aus dem Rind wurde zuerst von Chantry et al. (1989) (J. Biol. Chem. 264, Seiten 21603 bis 21607) gereinigt und von Howard et al. (1996) (Biochem. J. 317, Seiten 45 bis 50, GenBank/EMBL-Datenbank-Zugangsnummer Z21961) kloniert. Auch das Homologe des Menschen ist kloniert (Rosendahl et al. (1997), J. Biol. Chem. 272 (39), Seiten 24588 bis 24593, GenBank/EMBL-Datenbank-Zugangsnummer AF009615). Es war bisher unbekannt, was das natürliche Substrat beider Enzyme *in vivo* ist.
Es wurde nun gefunden, daß die Disintegrin-Metalloprotease ADAM 10, soweit bisher bekannt, alle Eigenschaften der α-Sekretase aufweist, wodurch es nun möglich ist, Zellen herzustellen, die α-Sekretase zusammen mit APP in großen Mengen exprimieren.
Die erfindungsgemäßen Zellen sind vorzugsweise Säugerzellen, die mit Vektoren, die Nukleinsäuresequenzen enthalten, die für das Substratprotein und für das Proteaseprotein codieren, auf bekannte Weise stabil oder transient transfiziert wurden (bezüglich Transfektion siehe Sambrook et al., Molecular Cloning, Cold Spring Harbor Laboratory Press, 1989, New York, USA). Es kann wahlweise ein einziger Vektor verwandt werden, der die Expression zweier Proteine ermöglicht (z.B. pIRESneo, Clontech, Palo Alto, California. USA) oder ein Gemisch aus zwei Vektoren, von denen jeder die Expression eines Proteintyps bewirkt. Alternativ können die Nukleinsäuren, die für das Substratprotein bzw. für das Proteaseprotein codieren, jeweils getrennt voneinander stabil oder transient transfiziert werden. Außerdem kommen virale Expressionssysteme, z.B. solche die auf SV40-Viren basieren, in Frage.
Ein anderes geeignetes Expressionssystem stellt die Injektion eines RNA-Gemisches in Xenopus Oocyten dar. In diesem Fall wird DNA, die für das erste bzw. zweite Protein codiert, in geeignete Vektoren kloniert, *in vitro*-transkribiert und als Transkriptgemisch in Xenopus Oocyten injiziert. In diesem Fall handelt es sich bei dem Begriff "rekombinante Nukleinsäure" um heterologe RNA. Der Begriff "heterologe RNA" bedeutet im Rahmen der Erfindung, daß die RNA sich von der endogenen RNA unterscheidet, da sie aus einem anderen Organisnus stammt. Üblich ist es, in Xenopus Oocyten RNA zu injizieren, die aus Säugerzellen stammt, also heterolog in bezug auf Xenopus ist.

Auch Pflanzenzellen sind schon erfolgreich als Wirtszellsystem eingesetzt worden (Lüring und Witzeman (1995) FEBS. Lett. 361 (1), Seiten 65 bis 69). Ferner kann es sich bei der Zelle auch um eine Hefe, eine Insektenzelle oder um eine Bakterienzelle handeln.

Die erfindungsgemäßen Zellen, weisen den Vorteil auf, daß die α-Sekretase in solch gleichbleibend hoher Konzentration gebildet werden kann, daß der Nachweis des Proteolyseproduktes in reproduzierbarer und einfacher Weise erfolgen kann.
Dieser Vorteil wird dadurch bewirkt, daß durch entsprechende Wahl der rekombinanten Nukleinsäure(n) die Menge an Transkripten, die für das Proteaseprotein codieren, gesteuert werden kann. In der erfindungsgemäßen Zellen wird das Proteaseprotein also unter Zuhilfenahme rekombinanter Methoden erzeugt.
Damit die Funktion der Protease verfolgt werden kann, müssen die erfindungsgemäßen Zellen ein Substratprotein exprimieren. Dies kann endogen erfolgen, das heißt der verwendete Zelltyp stellt natürlicherweise das Substratprotein über eine ausreichende Zeitspanne im Lebenslauf einer Zelle her, die die Beobachtung der Funktion der Protease zuläßt. Hierbei ist zu beachten, daß das Substratprotein von praktisch jeder Säugerzelle gebildet zu werden scheint. Insbesondere exprimieren auch Zellen aus Zelllinien wie HEK 293 ein Substratprotein (siehe Beispiel 8).

Auf besonders effektive Weise wird das Substratprotein durch rekombinante Methoden bereitgestellt. Hierdurch kann Substratprotein in weit größerer Menge produziert werden als dem endogenen Proteinspiegel entspricht (siehe Beispiel 11).

Es werden in der Regel Expressionsvektoren eingesetzt, die besonders starke Promotoren wie den Cytomegalie-Viruspromotor (CMV-Promotor) oder einen induzierbaren Promotor wie den MMTV-LTR-Promotor (Lee et al. (1981) Nature 294, Seite 228) besitzen. Diese Promotoren bewirken eine reproduzierbare und vergleichsweise starke Transkription der Gene, die für das Substratprotein bzw. das Proteaseprotein codieren.

Erfindungsgemäß ist es erforderlich, daß die rekombinate Zelle rekombinante Nukleinsäure(n) enthält, die mindestens ein für ein Proteaseprotein codierendes Gen aufweisen. Das Gen oder die Gene sind unter der Kontrolle eines heterologen Promotors, das heißt unter der Kontrolle eines Promotors, der die betreffenden Gene in ihrer natürlichen genetischen Umgebung nicht kontrolliert (Fremd-Promotor).

Ferner weisen die rekombinaten Nukleinsäure(n) zusätzlich ein Gen auf, das für ein Substratprotein codiert.
Umfaßt die rekombinante Nukleinsäure(n) Gene, die für ein Substratprotein und ein Proteaseprotein codieren, so stehen mindestens das für ein Proteaseprotein codierende Gen, vorzugsweise aber beide Gene, d.h. das Gen für das Substratprotein und das Gen für das Proteaseprotein, unter der Kontrolle eines heterologen Promotors.

Der Begriff Gen bezeichnet im Rahmen der Erfindung einen DNA-Abschnitt aus dem Genom einer Zelle, der für ein bestimmtes Protein oder Polypeptid codiert. Dieser Begriff erstreckt sich im Rahmen der Erfindung und abweichend vom normalen Sprachgebrauch auch auf die korrespondierende cDNA des Genomabschnitts. Die korrespondierende cDNA kann auf eine mRNA zurückgehen, die vollständig oder unvollständig gespleißt ist.

Andererseits kann durch die erwähnte Injektion *in vitro* transkribierter heterologer RNA in Xenopus Oocyten der Transkript-Spiegel direkt kontrolliert werden. Durch Steuerung des Transkriptspiegels gelingt eine Steuerung der Translation, also letztlich eine Steuerung der Menge an Genprodukt in der Zelle.

Bei dem Substratprotein handelt es sich entweder um das humane Amyloidvorläuferprotein (APP, amyloid precursor protein, Schilling et al. (1991) Gene 98, Seiten 225 bis 230, GenBank/EMBL-Datenbank-Zuganesnummer X06989, Y00297) oder um ein homologes Protein aus einem anderen Organismus, das über mindestens 770 Aminosäuren mit dem humanen Gegenpart einer Sequenzidentität von 86 % auf Aminosäureebene aufweist. Das Protein kann auch jeweils nur einen Teil des APPs umfassen, der mindestens die putative α-Sekretasespaltstelle, bestimmt durch Esch et al. (1990) Science 248, Seiten 1122 bis 1124, umfaßt sowie die Bereiche des APP, die an der Erkennung der Spaltstelle durch die α-Sekretase beteiligt sind (siehe z.B. Sisodia (1992) Proc. Natl. Acad. Sci. USA 89, Seiten 6075 bis 6079). Das Wort Spaltstelle oder Schnittstelle bezeichnet den zwischen zwei Aminosäuren einer Proteinsequenz gelegen Ort, an dem durch die Spaltung neue Termini entstehen. Durch Erzeugung von APP-Deletionsmutanten läßt sich auf einer dem Fachmann bekannten Weise die Erkennungsregion auf dem APP identifizieren. Das Substratprotein umfaßt somit in der Regel einen Sequenzbereich des APP oder eines Homologen, der die α-Sekretaseschnittstelle sowie die α-Sekretaseerkennungsregion beinhaltet. Hierbei wird ein Sequenzbereich von 6 Aminosäureresten, insbesondere von 36 Aminosäureresten aminoterminal und von 12 Aminosäureresten, insbesondere 83 Aminosäureresten carboxyterminal bezogen auf die α-Sekretaseschnittstelle bevorzugt. Im Prinzip sind aber alle Proteine geeignet, die den Sequenzbereich aufweisen, der an der Erkennung der Spaltstelle durch die α-Sekretase beteiligt ist.

Das Proteaseprotein umfaßt mindestens den für die proteolytische Aktivität notwendigen Sequenzbereich der Disintegrin-Metalloprotease ADAM 10 aus dem Rind (*Bos taurus*) oder aus dem Menschen oder eines homologen Proteins. Altenativ kann das Proteaseprotein auch durch Mutation (Insertion, Deletion oder Austausch von Aminosäuren) aus einer Säugetier Disintegrin-Metalloprotease ADAM 10 hervorgehen (Mutein bedeutet mutiertes Protein), solange die enzymatischen Eigenschaften im wesentlichen erhalten bleiben, das heißt denen des Wildtyps im wesentlichen entsprechen. Die Disintegrin-Metalloproteasen ADAM 10 aus Mensch und Rind sowie aus Maus und Ratte sind bevorzugt. Daneben fallen auch homologe Proteine unter die Definition des Proteaseproteins, die über 824 Aminosäuren mit der Disintegrin-Metalloprotease ADAM 10 aus dem Rind oder dem Menschen eine Sequenzidentität von 21 %, bevorzugt von 41 %, insbesondere von 61 %, vor allem von 81 %, am stärksten bevorzugt von 94 % auf Aminosäureebene aufweisen. Bevorzugt umfaßt das Proteaseprotein den Sequenzbereich von 213 bis 455, insbesondere von 213 bis 748, vor allem von 1 bis 748 bezogen auf die Disintegrin-Metalloprotease ADAM 10 des Rinds in der maximal langen Form (*full length*) bzw. den Sequenzbereich von 213 bis 455, insbesondere von 213 bis 748 vor allem von 1 bis 748 bezogen auf die Disintegrin-Metalloprotease ADAM 10 des Menschen in der maximal langen Form (*full length*).

Ein weiterer Gegenstand der Erfindung ist ein Verfahren zur Auffindung pharmazeutischer Wirksubstanzen.
Das erfindungsgemäße Verfahren ist gekennzeichnet durch die folgenden Schritte:
a) Bereitstellung einer erfindungsgemäßen rekombinanten Zelle,
b) Applikation einer Testsubstanz auf eine Zelle, auf eine Zellfraktion oder auf ein Gemisch von Zellfrak-tionen, die jeweils sowohl das Substratprotein als auch das Proteaseprotein umfassen, und
c) Quantitative Bestimmung eines seit der Applikation der Testsubstanz gebildeten Proteolyseproduktes des Substratproteins.

Durch das erfindungsgemäße Verfahren ist es möglich, auf einfache und reproduzierbare Weise den Effekt von pharmazeutischen Wirksubstanzen auf die α-Sekretaseaktivität zu testen und auf diese Weise nach pharmazeutischen Wirksubstanzen mit vorteilhaften Wirkungen zu suchen. Zu den vorteilhaften Wirkungen, die bei der Behandlung von neurodegenerativen Erkrankungen, insbesondere *Morbus Alzheimer* von Interesse sein könnte, gehört insbesondere die Stimualtion der α-Sekretaseaktivität der Disintegrin-Metalloprotease ADAM 10.
Ein weiterer Gegenstand der Erfindung ist somit die Verwendung von Substanzen, die die von der Zelle gebildete Proteinmenge oder die enzymatische Aktivität eines Proteaseproteins beeinflussen, zur Herstellung eines Medikaments zur Behandlung neurodegenerativer Erkrankungen, insbesondere von *Morbus Alzheimer.*

Die Bereitstellung eines Substratproteins und eines Proteaseproteins wird in der Regel durch zeitgleiche Coexpression beider Proteine in den geeigneten Wirtszellsystemen auf bekannte Art und Weise bewerkstelligt. Gegebenenfalls ist die Induktion der Expression notwendig (bei induzierbaren Promotoren wie MMTV-LTR). Wurden Zellen transient transfiziert oder RNA injiziert, so werden die Zellen zum Zeitpunkt der höchsten Expressionsdichte entnommen.
Das Verfahren kann mit intakten Zellen durchgeführt werden. In diesem Falle wird die Testsubstanz dem Kulturmedium zugegeben und die seit der Zugabe der Testsubstanz gebildete Menge an Proteolyseprodukt des Substratproteins im Zellüberstand bestimmt. Alternativ können die Zellen aufgebrochen werden, wobei die Testsubstanz einer Zellfraktion oder einer Mischung aus Zellfraktionen zugesetzt wird, die sowohl das Substratprotein als auch das Proteaseprotein umfaßt. Sollen Zellfraktionen eingesetzt werden, so ist es theoretisch auch möglich, Substratprotein und Proteaseprotein getrennt in zwei Zellinien zu exprimieren, die Zellen aufzubrechen und die Membranfraktionen zu vereinigen. Bei Einsatz von Zellfraktionen erfolgt die quantitative Bestimmung des seit der Applikation der Testsubstanz gebildeten Proteolyseproduktes nach Zentrifugation der Probe im Membranüberstand.

Ein weiterer Gegenstand der Erfindung ist die Verwendung eines erfindungsgemäßen Proteaseproteins zur proteolytischen Spaltung eines erfindungsgemäßen Substratproteins.

Ein weiterer Gegenstand der Erfindung ist eine rekombinante Zelle enthaltend eine rekombinante Nukleinsäure(n), die codieren:
a) für eine dominant negative Form der Disintegrin-Metalloprotease ADAM 10 aus dem Rind (Bos taurus) oder aus dem Menschen, wobei es sich bei der dominant negativen Form der Disintegrin-Metalloprotease ADAM 10 um die Mutante E384A der Disintegrin-Metalloprotease ADAM 10 aus dem Rind oder um eine Mutante aus dem Menschen mit derselben dominant negativen Funktion handelt, und
b) gegebenenfalls für ein Substratprotein wie zuvor definiert.

Dominant negative Formen der Disintegrin-Metalloprotease ADAM 10 können durch Punktmutation in einem besonders konservierten Bereich, insbesondere im aktiven Zentrum des Enzymes, erhalten werden. Ein Beispiel für eine dominant negative Form der Disintegrin-Metalloprotease ADAM 10 ist die Mutante E384A der Disintegrin-Metalloprotease ADAM 10 des Rindes, die in den Beispielen beschrieben und hier besonders bevorzugt ist. Entsprechende Mutanten aus Mensch oder einem anderen Säugetier sind ebenfalls bevorzugt. Die rekombinanten Zellen weisen den Vorteil auf, daß durch die Expression der dominant negativen Form die endogene α-Sekretaseaktivität fast vollständig ausgeschaltet, was sich in einer verminderten proteolytischen Spaltung des ebenfalls exprimierten Amyloidvorläuferproteins äußert. Dies erleichtert die Suche nach neuen Sekretasen.

Ein weiterer Gegenstand der Erfindung ist ein Verfahren zur Auffindung weiterer Sekretasen oder pharmazeutischer Wirksubstanzen, umfassend die folgenden Schritte
a) Expression eines Substratproteins und einer dominant negativen Form der Disintegrin-Metalloprotease ADAM 10 aus dem Rind (*Bos taurus*) oder aus dem Menschen in einer oben beschriebenen Zelle.
b) Expression eines weiteren Testproteins in der Zelle und/oder Applikation einer Testsubstanz auf die Zelle oder auf eine Zellfraktion dieser Zelle, und
c) Quantitative Bestimmung eines gebildeten Proteolyseproduktes des Substratproteins.

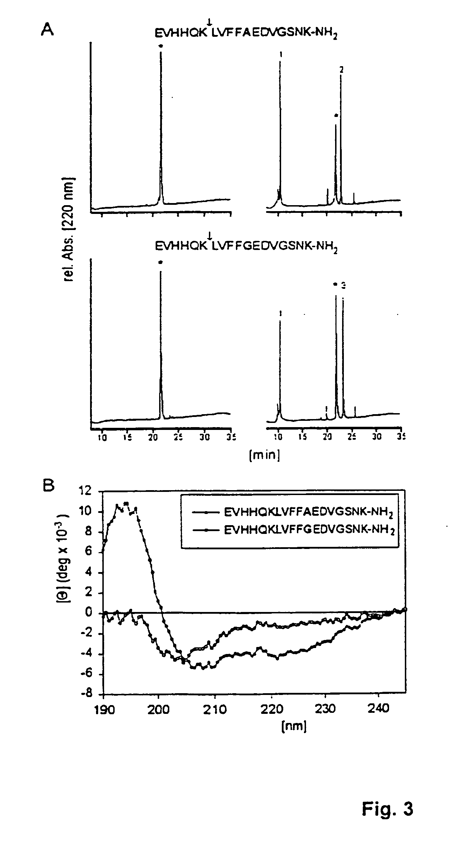

Dies Verfahren beruht auf der Erkenntnis, daß eine dominant negative Form der Disintegrin-Metalloprotease ADAM 10 die endogene α-Sekretaseaktivität einer Zelle, die dieses Protein exprimiert, unterdrückt. Erfindungsgemäß werden in diesem Verfahren nur Zellen oder Zellmembranen verwendet, die die dominant negative Form aufweisen.
Das Testprotein wird unter Anwendung der dem Fachmann bekannten Techniken in derselben Zelle exprimiert, die auch das Substratprotein und die dominant negative Form coexprimiert. Bei dem Verfahren wird geprüft, ob die Expression des Testproteins die Menge pro Zeiteinheit gebildeten Proteolyseprodukt des Substratproteins erhöht. Ist dies der Fall, so ist dies ein starker Hinweis darauf, daß es sich bei dem Testprotein um eine Sekretase handelt, die sich von der α-Sekretase bzw. von der Disintegrin-Metalloproteinase ADAM 10 unterscheidet. Durch Versuche mit künstlichen Peptiden (etwa gemäß Fig. 3) läßt sich klären, ob das Testprotein selber eine Protease/Sekretase ist oder die Aktivität einer Protease nur beeinflußt. Ferner kann eine Testsubstanz auf die Zelle oder auf eine Zellfraktion dieser Zelle gegeben werden, um festzustellen, ob die Testsubstanz die Aktivität der Sekretase, die keine α-Sekretase bzw. nicht die Disintegrin-Metalloproteinase ADAM 10 ist, beeinflußt.

Ein weiterer Gegenstand der Erfindung ist die Verwendung einer dominant negativen Form der Disintegrin-Metalloprotease ADAM 10 aus dem Rind (*Bos taurus*) oder aus dem Menschen oder einer Nukleinsäure, codierend für eine dieser dominant negativen Formen, zur Unterdrückung der α-Sekretaseaktivität von zellulären Testsystemen in vitro.

Bei einer bevorzugten Form einer erfindungsgemäßen Zelle codiert eine rekombinante Nukleinsäure ein Substratprotein, das ein Fusionsprotein aus einem Markerprotein oder einem Enzym und dem Amyloidvorläuferprotein darstellt. Der aminoterminale Sequenzbereich des Fusionsproteins wird von dem Markerprotein (wie GFP, *Green fluorescent Protein*), einem Enzym oder dem katalytisch wirksamen Teil eines Enzyms und der carboxyterminale Sequenzbereich vom Amyloidvorläuferprotein oder einem Teil des Amyloidvorläuferproteins gebildet.

Bei einer bevorzugten Form der erfindungsgemäßen Zelle handelt es sich bei dem Enzym um die alkalische Phosphatase SEAPs, in der gegenüber der Wildtyp-Variante SEAP die 24 carboxyterminalen Aminosäuren fehlen (SEAP : Soluble Human Secreted Alkaline Phosphatase). Diese SEAP-Variante ist verkürzt, um die Anheftung des Enzyms über GPI-Kopplung zu verhindern. Nukleinsäure, die für SEAP codiert, ist kommerziell erhältlich (pSEAP, Tropix Corp., Belford, Massachusetts, USA, Genbank-Zugangsnummer U09660).

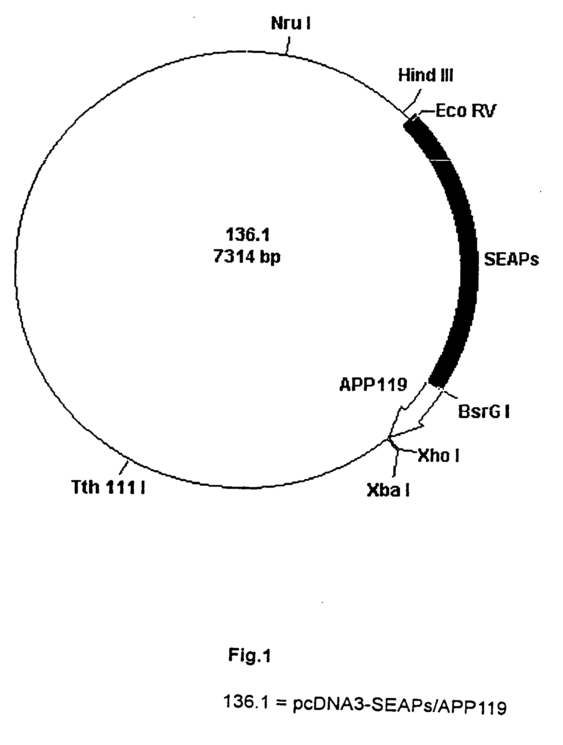

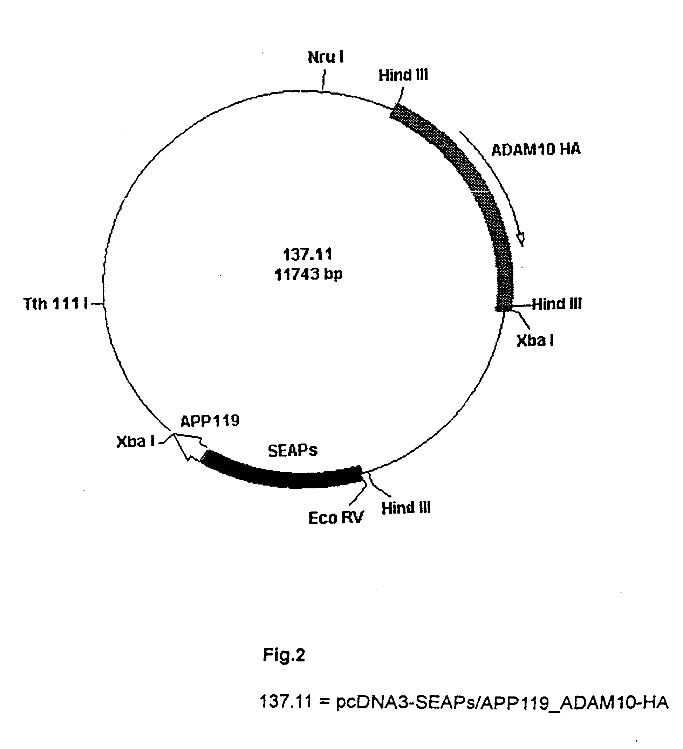

Bei einer bevorzugten Form der erfindungsgemäßen Zelle codiert einer rekombinante Nukleinsäure für ein Substratprotein, das eine Sequenz gemäß SEQ ID-No.:1 aufweist. Dieses Protein stellt ein Fusionsprotein aus SEAPs und den 119 carboxyterminalen Aminosäuren des humanen Amyloidvorläuferproteins (APP₁₁₉) dar. Dieses Protein wird z.B. von einer Nukleinsäure gemäß SEQ ID-No.:2 codiert. Diese Nukleinsäure kann Bestandteil eines Expressionsvektors sein z.B. gemäß Vektor 136.1 (pcDNA3-SEAPs/APP₁₁₉, siehe Fig. 1) oder gemäß Vektor 137.11 (pcDNA3-SEAPs/APP_{119_}ADAM10-HA, siehe Fig. 2).

Bei einer bevorzugten Ausführungsform eines der erfindungsgemäßen Verfahren erfolgt die quantitative Bestimmung des Proteolyseproduktes durch einen enzymatischen Test, bei dem von der katalytischen Aktivität der Probe auf die Menge an Proteolyseprodukt geschlossen wird. Dies Verfahren kann z.B. unter Verwendung von erfindungsgemäßen Zellen durchgeführt werden, die ein Substratprotein gemäß SEQ ID-No.:1 exprimieren. Durch die α-Sekretaseaktivität der Disintegrin-Metalloprotease ADAM 10 wird der aminoterminale Teil des Substratproteins abgespalten. Da dieses Proteolyseprodukt alkalische Phosphataseaktivität hat, läßt es sich im Zell- oder Membranüberstand z.B. durch Chemilumineszenz nachweisen. Hierbei können handelsübliche Testsysteme wie Phospha-Light^{tm} von Tropix Corp. (Belford, Massachusetts, USA) verwandt werden.
Die Abtrennung des Zellüberstands oder des Zellmembranüberstands nach Applikation der pharmazeutischen Wirksubstanz hat so zu erfolgen, daß sich die spezifische enzymatische Aktivität des Proteolyseproduktes durch den Abtrennvorgang nicht ändert oder sich in reproduzierbarer Weise ändert, so daß Proteolyseproduktmenge und katalytische Aktivität korreliert werden kann.

Bei einer weiteren bevorzugten Ausführungsform eines der erfindungsgemäßen Verfahren erfolgt die quantitative Bestimmung des Proteolyseproduktes durch einen spektroskopischen Test (wie Fluorometrie), bei dem von der spektroskopischen Eigenschaft einer Probe (wie Fluoreszenz) auf die Menge an einem bestimmten Proteolyseprodukt in der Probe geschlossen wird. Diese Ausftihrunesform bietet sich an, wenn das Substratprotein ein Fusionsprotein aus einem fluoreszeierenden Protein (wie GFP) und dem lunyloidvorläuferprotein darstellt.

Ein weiterer Gegenstand der Erfindung sind Verfahrenen zur Bestimmung des Risikofaktors für neurodegenerativen Erkrankungen, insbesondere für Morbus *Alzheimer.* Bei diesem Verfahren wird eine DNA-Probe der Testperson unter Einsatz genspezifischer Primer gegen die humane Disintegrin-Metalloprotease ADAM 10 der Polymerasekettenreaktionsanalyse (PCR-Analyse, PCR-Reakrtion) unterworfen, das Amplifikationsprodukt ggf. sequenziert und die Sequenz des Amplifikationsproduktes mit der bekannten Sequenz der humanen Disintegrin-Metalloprotease ADAM 10 verglichen. Die Anzahl und Position der Sequenzunterschiede, die sich in einer anderen Aminosäureabfolge äußert, läßt Rückschlüsse auf den Risikofaktor für neurodegenerativen Erkrankungen, insbesondere für *Mobus Alzheimer* zu. Hierbei wird angenommen, daß Sequenzunterschiede, die zu einer Disintegrin-Metalloprotease ADAM 10-Variante mit beeinträchtigter α-Sekretaseaktivität führen, eine Prädisposition für neurodegenerativen Erkrankungen, insbesondere für *Morbus Alzheimer* zur Folge haben. Ferner kann das Amplifikationsprodukt einer Schmelzpunktanalyse unterzogen und auch auf diese Weise mit der bekannten Sequenz verglichen werden. Für diesen Zweck werden Temperaturgradienten-Gelelektrophorese und fluoreszenzspektroskopische Methoden eingesetzt (z.B. TGGE-System der Fa. Biometra, LiahtCycler^{™} der Fa. Hoffmann-LaRoche). Darauf aufbauend können spezifische Gensonden gegen humanes ADAM 10 oder dessen mutierte Varianten für diagnostische Zwecke eingesetzt werden (z.B. im Rahmen der FISH-Technik (*fluorescent in situ hybridisation*) oder mit Hilfe von DNA-Chips). Als Gensonden kommen Nukleinsäure- oder Peptid-Nukleinsäureoligomere in Betracht.
Ein weiterer Gegenstand der Erfindung ist somit ein Verfahren zur Bestimmung des Risikofaktors für neurodegenerative Erkrankungen, insbesondere für *Morbus Alzheimer,* wobei
a) eine DNA-Probe der Testperson unter Einsatz genspezifischender Primer gegen die humane Disintegrin-Metalloprotease ADAM 10 der PCR-Analyse unterworfen wird,
b) und die Sequenz des Amphfikationsproduktes mit der bekannten Sequenz der humanen Disintegrin-Metalloprotease ADAM 10 verglichen wird.

Mit Hilfe von Gensonden wurde der chromosomale Lokus von humanem ADAM 10 auf Chromosom 15 bei 12.44 cR3000 zugeordnet (Yamazaki et al. (1997) Genomics 45, Seiten 457-459). Durch chromosomale Translokation oder auch durch Alleldeletion in Chromosomen (Bsp. Tumorsuppressor p53) kann es zu einem Aktivitätsverlust von Genprodukten kommen. Es ist daher nicht auszuschließen, daß neurodegenerative Erkrankungen, insbesondere *Morbus Alzheimer* durch einen Aktivitätsverlust der ADAM 10-Protease bedingt wird, welcher aus einer chromosomalen Veränderung resultiert. Durch Verwendung von ADAM 10-Gensonden ließe sich ein Fehlen oder eine falsche Lokalisierung des ADAM 10-Gens bestimmen und als diagnostischer Parameter einer Prädisposition für neurodegenerativen Erkrankungen, insbesondere für *Morbus Alzheimer* nutzen. Ein weiterer Gegenstand der Erfindung ist somit ein Verfahren zur Bestimmung des Risikofaktors für neurodegenerative Erkrankungen, insbesondere für *Morbus Alzheimer,* wobei die tatsächliche chromosomalen Lokalisierung des Gens der Disintegrin-Metalloprotease ADAM 10 mit seiner normalen chromosomalen Lokalisierung, Chromosom 15 bei 12.44 cR₃₀₀₀, verglichen wird.

Eine Verringerung der Aktivität der ADAM 10-Protease kann darüber hinaus durch eine verminderte Transkription des ADAM 10-Gens bedingt werden. Die Effizienz der Transkription des ADAM 10-Gens läßt sich durch die Quantifizierung der ADAM 10-mRNA ermitteln und als Parameter bei der Diagnose von neurodegenerativen Erkrankungen, insbesondere von *Morbus Alzheimer* oder auch zur Diagnose einer Prädisposition dieser Krankheiten nutzen. Dazu muß die ADAM 10-mRNA aus Zell- oder Gewebeproben isoliert und quantifiziert werden. Zur direkten Quantifizierung der mRNA bietet sich die sogenannte Northern-Blot-Analyse an. Eine z.B. radioaktiv markierte, zur ADAM 10-mRNA komplementäre Nukleinsäuresonde hybridisiert dabei mit der ADAM 10-mRNA. Die Menge an spezifisch gebundener Sonde wird anschließend, nach den dem Fachmann bekannten Methoden, quantifiziert. Die ADAM 10-mRNA kann nach bekannten Methoden zunächst in einzelsträngige, komplementäre DNA und dann in doppelsträngige komplementäre DNA (cDNA) umgewandelt werden. Sowohl die einzelsträngige als auch die doppelsträngige DNA läßt sich durch dem Fachmann bekannte Methoden, wie z.B. Echtzeit-PCR, DNA-Mikro-Array-Analyse, DNA Chiptechnologie etc., quantifizieren.

Ein weiterer Gegenstand der Erfindung ist somit ein Verfahren zur Bestimmung des Risikofaktors für neurodegenerative Erkrankungen, insbesondere für *Morbus Alzheimer,* durch quantitative Bestimmung der mRNA-Mengen von humanem ADAM 10 aus Zell- und Gewebeproben, umfassend einen der folgenden Schritte:
a) Isolierung und Quantifizierung der ADAM 10-mRNA,
b) Einzel- oder doppelsträngige cDNA die ADAM 10-mRNA umgeschrieben wird welche dann quantifiziert wird,
c) die ganze Nukleotidsequenz von ADAM 10 oder Teile davon zur Quantifizierung mittels Hochdurchsatz-Expressionsanalyse (z.B. DNA-ChipTechnologie u. DNA-Micro-Array) eingesetzt wird.

Die α-Sekretaseaktivität von ADAM 10 läßt sich durch Aktivatoren der Proteinkinase C stimulieren. ADAM 10 besitzt in seinem Carboxylterminus einen Threoninrest in Aminosäureposition 719 (T719), welcher eine Proteinkinase C-Phosphorylierungsstelle darstellt. Es ist naheliegend, daß die Aktivität von ADAM 10 durch Phosphorylierung an Threonin 719 gesteigert wird. Für den Aktivitätsverlust von ADAM 10 könnte daher eine fehlende, oder zumindest eine verminderte, Phosphorylierung von ADAM 10 verantwortlich sein. Methoden, mit denen der Phosphorylierungsgrad von ADAM 10 bestimmt werden kann, können daher zur Diagnose von neurodegenerativen Erkrankungen, insbesondere von *Morbus Alzheimer* und zur Diagnose einer Prädisposition dieser Kranheiten angewendet werden. Die Quantifizierung des Phosphorylierungsgrades von ADAM 10 kann an Zell- oder Gewebeproben durchgeführt werden oder auch an aus diesen Proben isoliertem ADAM 10 erfolgen. Durch zweidimensionale Polyacrylamid-Gelelektrophorese und anschließenden Elektrotransfer kann ADAM 10, getrennt von anderen Proteinen einer Zell- oder Gewebeprobe, auf proteinbindenden Membranen dargestellt werden. Die Phosphorylierung von ADAM 10 läßt sich anschließend, gegebenenfalls nach Tryptischem-Verdau, durch Massenspektrometrie, durch Anti-Phosphothreonin-Antikörper oder durch noch zu entwickelnde Anti-Phospho-ADAM 10-Antikörper quantifizieren. Mit den gleichen Methoden läßt sich auch der Phosphorylierungsgrad von gereinigtem ADAM 10 ermitteln. Es können auch Experimente herangezogen werden, mit denen man die Menge an Phosphothreonin bestimmt, bzw. bei denen man eine Dephosphorylierung von Phospho-ADAM 10 nach bekannten Methoden quantitativ durchführt (Ausubel et al. (Hrsg.) (1995) Current Protocols in Molecular Biology, John Wiley & Sons, New York).

Ein weiterer Gegenstand der Erfindung ist somit Verfahren zur Bestimmung des Aktivitätszustands von humanem ADAM 10 durch Quantifizierung des Phosphorylierungsgrades von humanem ADAM 10 in Zell- und Gewebeproben, wobei entweder
a) ADAM 10 isoliert wird und sein Pbosphorylierungsgrad entweder durch Massenspektrometrie, durch spezifische Anti-Phosphothreonin-Antikörper, durch Quantifizierung der Aminosäure Phosphothreonin oder durch Dephosphorylierung (Phosphatase-Assay) von ADAM 10 bestimmt wird, oder
b) Zell- oder Gewebeproben direkt in eine Proteomanalyse eingesetzt werden und der Nachweis von phosphoryliertem ADAM 10 wie unter Punkt a) beschrieben erfolgt.

Zum generellen Nachweis und zur Quantifizierung von ADAM 10 in Zell- und Gewebeproben bieten sich darüber hinaus spezifische, ADAM 10 erkennende Antikörper und Antikörperderivate an. Unter Antikörperderivaten werden hier rekombinante Einzelketten-Antikörper und auch rekombinante Phagen verstanden, die rekombinante Einzelketten-Antikörper enthalten. Spezifisch ADAM 10 erkennende Antikörper können daher zur Diagnose von neurodegenerativen Erkrankungen, insbesondere von *Morbus Al_{z}heimer* und zur Feststellung einer Prädisposition für neurodegenerativen Erkrankungen, insbesondere für *Morbus Alzheimer* verwendet werden.

Ein weiterer Gegenstand der Erfindung ist also die Verwendung von Antikörpern oder Antikörperderivaten, die ein Proteaseprotein spezifisch binden, zum quantitativen in vitro - Nachweis der alpha-Sekretase und/oder zur in vitro-Diagnose einer Prädisposition zur Ausbildung der Alzheimerschen Krankheit.

Folgende transgene nicht-meschliche Tiere sind weitere erfindungsgemäße Gegenstände.
a) Transgenes nicht-menschliches Tier, das *eine* rekombinate Nukleinsäure, codierend für eine dominant negative Form der Disintegrin-Metalloprotease ADAM 10 aus dem Rind (*Bos taurus*) oder aus dem Menschen, wie oben beschreiben, aufweist und exprimiert.

Bevorzugt wird das erfindungsgemäße nicht menschliche transgene Tier als in vivo-Modell eines Organismus mit einer Prädisposition zur Ausbildung der Alzheimerschen Krankheit zur Identifikation und Charakterisierung von neuen Arzneimittel-Wirkstoffen gegen die Alzheimersche Krankheit verwendet.
Ebenso bevorzugt wird das erfindungsgemäße nicht-menschliche transgene Tier zur Identifikation und Charakterisierung neuer Proteasen mit alpha-Sekretaseaktivität verwendet.

Transgene Tiere können im Rahmen der Erfindung homozygot oder heterozygot in bezug auf das Gen sein, das dem Tier die transgene Eigenschaft verleiht. Außerdem sind von dem Begriff "transgenes Tier" Menschen nicht umfaßt.
Transgene Tiere werden auf an sich bekannte Weise erzeugt. Hierbei wird bei einer Ausführungsform eine rekombinante Nukleinsäure verwendet, z.B. in Form eines Vektors, die für ein Proteaseprotein codiert. Bei einer anderen wird rekombinante Nukleinsäure verwendet, z.B. in Form eines Vektors, die für eine dominat negative Form der Disintegrin-Metalloprotease ADAM 10 aus dem Rind (*Bos taurus*)*,* aus dem Menschen codiert.

Die nicht-menschlichen transgenen Tiere können als Tiermodelle für die Suche und die Erprobung von pharmakologischen Wirkstoffen gegen neurodegenerativen Erkrankungen, speziell gegen *Morbus Alzheimer* verwendet werden Weiterhin können physiologische Veränderungen oder Verhaltenseigenarten der Tiere Ausschluß darüber geben, welche Prozesse oder Entwicklungsstadien eines Organismus durch ADAM 10 beeinflußt werden.

Die rekombinaten Nukleinsäure, codierend für ein Proteaseprotein oder für eine dominant negative Form der Disintegrin-Metalloprotease ADAM 10 aus dem Rind *(Bos taurus)* oder aus dem Menschen kann zur Erzeugung transgener Zellen und transgener nicht-menschlicher Tiere und zur Gentherapie verwendet werden.

Die Verwendung einer Nukleinsäure, die für die Disintearin-Metalloprotease ADAM 10 des Menschen codiert, ist bevorzugt. Bei der Konstruktion des Vektors für die Gentherapie wird die Nukleinsäure gegebenenfalls über eine Mehrfachschnittstelle (*multiple cloning site*) in den Vektor so eingebaut, daß in einer Zelle, die mit dem so konstruierten Vektor transfiziert ist, Expression der rekombinanten Disintearin-Metalloprotease ADAM 10 feststellbar ist. Durch das Einschleusen von Disintegrin-Metalloprotease ADAM 10 oder eines katalytisch aktiven Teils derselben in die Zielzellen im Rahmen der Gentherapie können α-Sekrctaseaktivitätsdefekte von Zellen kompensiert werden. Dadurch können die Symptome von *Morbus Alzheimer* und anderer neurodegenerativer Erkrakungen zurückgedrängt werden.

Ebenfalls zur Erfindung gehört also auch die Erzeugung von transgenen Tieren, bei denen eine ADAM 10-Protease, eine dominant negativ wirkende Mutante derselben, oder eine zur ADAM 10-DNA-Sequenz entgegengesetzt komplementäre (revers komplementäre) Nukleinsäure zusätzlich exprimiert bzw. transkribiert wird. Die zu transkribierende Nukleotidsequenz kann dabei unter Kontrolle eines ubiquitär aktiven Promotors wie z.B. dem Cytomegalie-Viruspromotor (CMV-Promotor) stehen. Mit diesem ist eine Expression in allen Geweben möglich. Alternativ kann die zu transkribierende Nukleotidsequenz unter Kontrolle eines gewebsspezifischen Promotors stehen. Von besonderem Interesse sind hier solche Promotoren, die eine gehirnspezifische Expression erlauben, insbesondere solche, die für Neuronen und Gliazellen spezifisch sind. Unter Verwendung des Promotors des Thy1-Glykoproteins der Maus gelang es beispielsweise, das humane Amyloid-Vorläuferprotein in Neuronen von Mäusen zu exprimieren (Moechars et al. (1999) J. Biol. Chem. 274, Seiten 6483-6492). In Analogie hierzu könnte eine ADAM 10-Protease, deren dominant negative Form oder eine entgegengesetzt komplementäre Form in Neuronen von Mäusen entweder alleine oder zusammen mit Varianten des humanen Amyloid-Vorläuferproteins exprimiert werden. Solche transgenen Tiere können als Modellsysteme zum Testen und Auffinden von Pharmaka und zur Untersuchung der Amyloidprozessierung verwendet werden.

Die Überexpression einer proteolytisch aktiven ADAM 10-Protease sollte dabei die Entstehung von Amyloid-β-Peptiden verhindern bzw. zumindest verlangsamen. Die Expression einer dominant negativ wirkenden ADAM 10-Mutante sollte die Aktivität der endogenen ADAM 10-Protease hemmen und damit zu einer vermehrten Entstehung von Amyloid-β-Peptiden beitragen. Eine zweite Strategie zur Ausschaltung der endogenen ADAM 10-Protease stellt die Transkription einer zur ADAM 10-cDNA entgegengesetzt komplementären DNA dar. Mit dieser sogenannten *antisense*-Strategie ist es möglich, die Expression eines endogenen Proteins in Zellkultur zu unterdrücken (siehe z.B. Volonte et al. (1999) FEBS Lett. 445, Seiten 431-438). Das Ausschalten von Genen ist mit einem solchen Ansatz auch in vivo möglich (siehe z.B. Hayashi et al. (1996) Gene Therapie 3, Seiten 878-885).

Ein weiter Gegenstand der Erfindung ist ein Arzneimittel, das eine rekombinante Nukleinsäure enthält, die für ein erfindungsgemäßes Proteaseprotein codiert. Ein solches rekombinantes Arzneimittel ist z.B. ein Virus oder ein Vektor für die Gentherapie, der Nukleinsäure, die für ein erfindungsgemäßes Proteaseprotein codiert, aufweist.

Als Arzneimittel kann weiterhin ein katalytisch aktiver, löslicher Proteinanteil von ADAM 10 dienen. Dieses Polypeptid wird nach den dem Fachmann bekannten Methoden rekombinant z.B. in Bakterien, Insektenzellen, Hefen oder eukaryontischen Säugerzellen hergestellt und in reiner Form isoliert. Die Applizierung dieses ADAM 10-Polypeptids erfolgt bevorzugt durch Injektion oder durch Resorption.

Die Erfindung wird durch die nachfolgende Zeichnung näher erläutert.

Es zeigt
- Fig. 1: den Expressionsvektor 136.1, der für ein Substratprotein codiert, das ein Fusionsprotein aus SEAPs und APP₁₁₉ darstellt.
- Fig. 2: den Expressionsvektor 137.11, der außer für das vorgenannte Fusionsprotein für ein Proteaseprotein codiert, welches aus einer markierten Disintegrin-Metalloprotease ADAM 10 besteht.
- Fig. 3: die proteolytische Spaltung von Peptiden, die die α-Sekretaseschnittstelle von APP aus dem Rind umfassen, durch Disintegrin-Metalloprotease ADAM 10.
- Fig.4: die Expression und Deglycosillerung der Disintegrin-Metalloprotease ADAM 10.
- Fig. 5: die Sekretion von APPsα von unveränderten HEK-Zellen (HEK 293, kurz HEK steht für *human embryonic kidney*) und von HEK-Zellen, die die Disintegrin-Metalloprotease ADAM 10 exprimieren (HEK-ADAM 10-Zellen).
- Fig. 6: den Einfluß des Metalloprotease ADAM 10 auf die Entstehung der Protolyseprodukte APPsα und p10.
- Fig. 7: den inhibierenden Effekt der dominant negativen Disintegrin-Metalloprotease ADAM 10 (DN).

Fig. 1 zeigt den Vektor 136.1, der auf einem pcDNA3-Vektor (Invitrogen BV, Groningen, Niederlande) basiert und in den eine Nukleinsäure einkloniert wurde, die für ein Protein gemäß SEQ ID-No.:1 codiert. Zur Konstruktion der Nukleinsäure wurde der Bereich, der die ersten 506 aminoterminalen Aminosäuren von SEAP codiert, durch PCR amplifiziert (Produkt: SEAPs-DNA). Als Matrize wurde der Vektor pSEAP (Tropix Corp., Belford, Massachusetts, USA) benutzt. Durch PCR wurde eine BsrGI-Restriktionsschnittstelle eingeführt. Die codierende Region für die letzten 119 carboxyterminalen Aminosäuren des menschlichen Amyloidvorläuferproteins wurden durch PCR auf der korrespondierenden cDNA amplifiziert (Produkt: APP₁₁₉-DNA). Durch PCR wurde eine BsrGI-Schnittstelle am 5'-Ende der cDNA eingeführt. Die BsrGI-Schnittstellen der SEAPs-DNA und APP₁₁₉-DNA wurden für die Konstruktion einer Nukeinsäure, die für das SEAPs/APP₁₁₉-Fusionsprotein gemäß SEQ ID-No.:1 codiert, eingesetzt.

Fig. 2 zeigt den Vektor 137.11, der Sequenzbereiche enthält, die für das vorgenannte Fusionsprotein und eine mit einem Marker (Influenza Hämagglutinin (HA) Epitop tag) markierte Disintegrin-Metalloprotease ADAM 10-Variante codieren. 137.11 basiert auf dem Vektor pcDNA3 der Firma Invitrogen.

Fig. 3 zeigt die proteolytische Spaltung von Peptiden, die die Stelle der α-Sekretasespaltung von APP durch ADAM 10 umspannen.
(A) Ordinate: relative Absorption [220 nm], Absisse: Zeit [min]. Peptidsubstrate wurden in Spaltungspuffer in Abwesenheit (linker Graph) oder in Anwesenheit von ADAM 10 (rechter Graph) 6 Stunden bei 37°C inkubiert und anschließend der HPLC-Analyse unterzogen. Die Sterne kennzeichnen die Peptidsubstrate und die Zahlen, die generierten Produkte: 1 : EVHHQK-OH; 2 : LVFFAEDVGSNK-NH₂; 3 : LVFFGEDVGSNK-NH₂.
(B) CD-Spektren von APP-Peptiden. Ordinate: Elliptizität [θ] (des x 10⁻³); Absisse: Wellenlänge [nm]. Die Peptide wurden in 20 mM Tris/HCl pH 8,3, 250 mM NaCl bei einer Endkonzentration von 0,5 mM gelöst. Die Messungen wurden in Gegenwart von 0,5 % Natriurndodecylsulfat (SDS) ausgeführt.

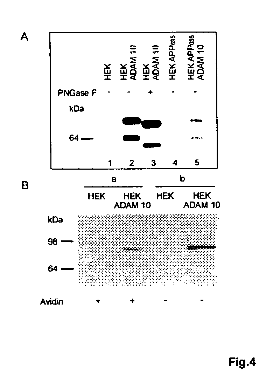

Fig. 4 zeigt die Expression und Deglycosilierung der Disintegrin-Metalloprotease ADAM 10
(A) nach Transfektion von HEK-Zellen und HEK-APP₆₉₅-Zellen mit ADAM 10-cDNA wurden Zellysate mit monoklonalen Anti-HA-Antikörper immunoprezipitiert, der Deglycosilierung mit PNGase F (Spur 3) unterworfen oder direkt durch ein 4-12 % NuPAGE-Gelsystem und Western Blot analysiert. HEK-APP₆₉₅-Zellen sind HEK-Zellen, die ein humanes 695 Aminosäuren großes Amyloidvorläuferprotein (GenBank/EBML-Datenbank-Zugangsnummer: Y00264) exprimieren. Immunoblots wurden mit dem ECL-Detektionssystem (Pharmacia&Upjohn GmbH, Erlangen) durchgeführt.
(B) HEK und HEK-ADAM-10-Zellen wurden mit 0,3 mg/ml Sulfo-NHS-LC-Biotin (Pierce, Rockford, Illinois, USA) in PBS 30 min bei 4°C inkubiert. Nach Immunopräzipitation mit dem monoklonalen Anti-HA-Antikörper (16B12) und Elution des Immunkomplexes wurden 4/5 des Eluats mit Streptavidin (a), inkubiert, während das verbliebene Fünftel direkt einer 10 %-igen SDS-PAGE unterworfen wurde (b) und anschließend auf PVDF-Membranen geblottet wurde. Die Detektion wurde wie oben beschrieben durchgeführt.

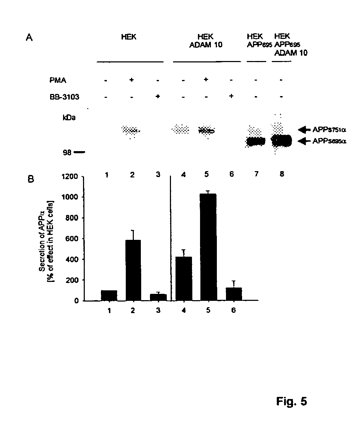

Fig. 5 zeigt die Sekretion von APPsα von HEK- und HEK-ADAM-10-Zellen.
(A) Die Zellen wurden in Gegenwart der bezeichneten Verbindung inkubiert. Nach 4 Stunden wurde das Medium gesammelt, die Proteine präzipitiert und einer Immunoblotanalyse mit dem monoklonalen Antikörper 6E10, gefolgt von einem sekundären [³⁵S]-markierten Anti-Maus-IgG-Antikörper, unterzogen.
(B) Quantitative Analyse des sekretierten APPsα. Die APPsα entsprechenden radioaktiven Banden wurden mit Hilfe eines Bio-Imaging Analyzer BAS-1800 quantifiziert. Die Meßwerte wurden wie in dem Beispiel beschrieben, auf verschiedene Proteinmengen normiert. Die Ergebnisse wurden als Prozentwerte von sekretiertem APPsα in nicht-rekombinanten Vergleichs-HEK-Zellen ausgedrückt und die Standardabweichungen aus mindestens drei unabhängigen Experimenten angegeben. Ordinate: Sekretion von APPα [Prozentwert der Wirkung in HEK-Zellen]. Absisse: Probennummer, dieselbe Numerierung wie in (A).

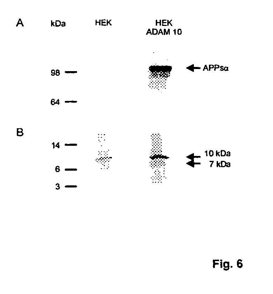

Fig. 6 zeigt den Einfluß der Disintegrin-Metalloprotease ADAM 10 auf die Entstehung der Proteolyseprodukte APPsα und p10.
(A) HEK und HEK-ADAM-10-Zellen wurden mit 200 µCi/ml [³⁵S] L-Methionin und Cystein fünf Stunden metabolisch markiert. Die Kulturmedien wurden mit dem polyklonalen Antikörper 1736 immunoprezipitiert und einer 10 %-igen SDS-PAGE unterzogen. Die Analyse wurde mit dem Bio-Imaging Analyzer BAS-1800 durchgeführt. APPs₇₅₁α bezeichnet das Proteolyseprodukt, das durch α-Sekretase-Einwirkung auf eine APP-Variante, welche 751 Aminosäuren umfaßt, entsteht.
(B) Die Zellysate wurden mit dem Antikörper C 7 immunoprezipitiert. Die Proben wurden mit Hilfe eines 10 - 20 %-igen Tris-Tricine Gel (Novex) getrennt und wie oben beschrieben analysiert.
(C) Quantitative Analyse von intaktem APP, das die ganze Länge aufweist (Holo-APP), und p10. Die entsprechenden radioaktiven Banden wurden wie in Fig. 5 quantifiziert. Die Werte von p10 wurden auf die Holo-APP-Spiegel normiert. Die Ergebnisse vier unabhängiger Experimente wurden mit den entsprechenden Standardabweichungen angegeben. Die statistische Signifikanz zwischen Vergleichszellen und HEK-ADAM-10-Zellen wurde mit Hilfe des ungepaarten Student's *t*-Test bestimmt (*, P<0,005).

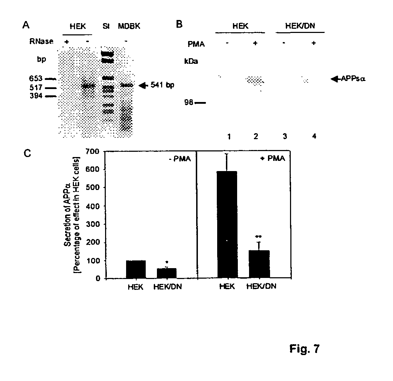

Fig. 7 zeigt den inhibierenden Effekt der dominant negativen Mutante DN der Disintegrin-Metalloprotease ADAM 10.
(A) Endogene Expression der Disintegrin-Metalloprotease ADAM 10 in HEK-Zellen. ADAM 10 mRNA von MDBK und HEK-Zellen wurde mit einem Oligonucleotid, das spezifisch für die bovine und die humane ADAM 10 mRNA ist, revers transkribiert und anschließend amplifiziert, was Fragmente mit 541 Basenpaaren ergab. Als Vergleich wurden HEK-Zellen, die vor dem Reverse-Transkriptase-Schritt mit RNase A behandelt worden waren, eingesetzt. Als Standard wurde der DNA-Molekulargewichtsmarker VI (St, siehe Abbildung) von Boehringer Mannheim eingesetzt. Die Fragmente zwischen 1230 und 154 Basenpaaren sind gezeigt.
(B) HEK und HEK/DN-Zellen wurden in Abwesenheit und Anwesenheit von 1 □M PMA inkubiert. HEK/DN-Zellen sind Zellen, die die dominant negativen Mutante DN der Disintegrin-Metalloprotease ADAM 10 exprimieren. Nach vier Stunden wurde das Medium gesammelt, die Proteine präzipitiert und einer Immunoblotanalyse, wie in Fig. 5 beschrieben, unterzogen.
(C) Quanitative Analyse des sekretierten APPsα. Die radioaktiven Banden gehören zu APPsα und wurden quantifiziert, wie in Fig. 5 beschrieben. Die Ergebnisse sind als Prozentwerte des sekretierten APPsα in Vergleichs-HEK-Zellen ausgedrückt und als Mittelwerte von mindestens drei unabhängigen Experimenten angegeben. Die Fehlerbalken beziehen sich auf die Standardabweichungen. Die statistische Signifikanz zwischen Kontrollzellen und HEK/DN-Zellen, mit und ohne PMA-Behandlung, wurde durch den ungepaarten Student's *t*-Test bestimmt (*, P<0,005; **, P<0,001).
   Ordinate: Sekretion von APPα [Prozentwert der Wirkung in HEK-Zellen].

Die Erfindung wird näher durch die nachfolgenden Beispiele beschrieben.

### Materialien

Die Peptide wurden mit dem Festphasenverfahren unter Verwendung von Fmoc-Chemie auf dem 9050 + Millipore Peptidsynthesizer synthesiert. BB-3103 wurde von der British Biotech zur Verfügung gestellt. Die Antikörper und ihre Quellen waren: Anti-HA-Epitop-IgG (monoklonaler Antikörper Klon 16B12 aus der Maus, BAbCO; polyklonaler Antikörper Y-11 aus dem Kaninchen, Santa Cruz Biotechnology), Anti-Kanninchen-IgG-Meerrettichperoxidase-gekoppelter Antikörper und [³⁵S] anti-Maus-IgG-Antikörper von Amersham Pharmacia Biotech. Antikörper gegen APP: monoklonaler Antikörper IgG 6E10 aus der Maus von Senetek: polyklonaler Antikörper 1736 aus dem Kaninchen und C7 wurden von Dennis J. Selkoe zur Verfügung gestellt. Weitere Chemikalien sind in Tabelle 1 aufgeführt. Sofern nicht anders gekennzeichnet sind sämtliche Lösungen wässrig.

**Tabelle 1:**

| | |
|---|---|
| **CHAPS:** | 3-[(3-Cholamidopropyl)Dimethylammonio]-1-Propansulfonat |
| **EPPS:** | N-[2-Hydroxyethyl]Piperazin-N'-[3-Propansulfonsäure] |
| **PBS:** | 8g/l NaCl; 0,2 g/l KCl, 1,44 g/l Na₂HPO₄; 0,24 g/l KH₂PO₄; pH 7,0-7,5 |
| **PMA:** | Phorbol-12-myristat-13-acetat |
| **TBS** | 8g/l NaCl; 0,2 g/l KCl; 3g/l Tris/HCl; pH 7,0-7,5 |
| **TCA** | Trichloressigsäure |
| **TFA** | Trifluoracetat |
| **Tris** | Tris(Hydroxymethyl)Aminomethan |

### Beispiel 1

### Reinigung der Disintegrin-Metalloprotease ADAM 10:

Die Disinmtegrin-Metalloprotease ADAM 10 wurde aus Rindernierenplasmamembranen nach dem Verfahren von Howard und Glynn (Meth. Enzymology (1995) 248, Seiten 388 bis 395) bis zur Homogenität aufgereinigt. Nach dem DEAE-Sephacel-Schritt wurden aktive Fraktionen direkt auf eine Hydroxylapatitsäule aufgegeben, die mit 20mM Tris/HCl pH 7, 0,1 mM K₂HPO₄ und 0,3 % CHAPS äquilibriert worden war. Aktives Enzym wurde mit 51 mM Kaliumphosphat pH 7,0 eluiert. Eine Analyse Enzymaktivität enthaltener Proteinfraktionen mit SDS-PAGE ergab ein Protein mit einem Molekulargewicht von etwa 62.000. Aminoterminale Sequenzanalyse ergab eine Aminosäuresequenz von TTVXEKNTXQLYIQTDXXFF, die identisch mit der von Rinder-MADM ist (Howard et al. (1996); Biochem. J. 317, Seiten 45 bis 50) ebenfalls bekannt als ADAM 10 (Wolfswerk et al. (1995) J. Cell Biol. 131, Seiten 275 bis 278).

### Beispiel 2

### Proteasetestverfahren zur Bestimmung der Substratspezifität:

Peptide einer Endkonzentration von 0,3 mM wurden mit 2 µg gereinigten Enzym in 50 µl 50 mM EPPS pH 8,3, 250 mM NaCl bei 37°C 30 Minuten oder 6 Stunden inkubiert. Die Reaktionen wurden mit 2 µl TFA gestoppt. Meßproben wurden mit Hilfe von HPLC auf einer Vydac (The Separation Group, Hesperia, Calif. USA) RP 18 Säule (5 µm, 250 x 4,6 mm) analysiert. Die Peptide wurden mit einem Puffersystem von 0,1 % TFA in Wasser (Puffer A) und 0,1 % TFA, 9,9 % Wasser, 90 % Acetonitril (Puffer B) aufgetrennt, wobei der folgende Gradient angelegt wurde: 0 % B, 5 Min.; 0 bis 70 % B innerhalb 45 Min. Das Molekulargewicht der Proteolyseprodukte wurde mit ESI-Massenspektrometrie bestimmt. Für die Berechnung der prozentualen Spaltung wurde die Peakfläche, die ein Peptid ergab, das ohne Enzym inkubiert und ansonsten identisch behandelt wurde, als Referenz für 100 % herangezogen.

### Beispiel 3

### Circulardichroismusspektoskopie:

CD-Messungen wurden auf einem Jasco J 720 Spek-tropolarimeter bei 4°C durchgeführt. Die Schichtdicke der Quarzmeßzelle betrug 0,1 mm. Die Messungen wurden bei einer Peptidkonzentration von 0,5 mM in 20 mM Tris/HCl bei pH 8,3, 250 mM NaCl in Anwesenheit von 0,5 % (w/v) SDS durchgeführt. Als Ergebnis wird die Elliptizität [θ] (deg x 10⁻³) aufgeführt.

### Beispiel 4

### Klonierung und funktionelle Expression der Disintegrin-Metalloprotease ADAM 10:

PCR-Primer, die auf die Nukleotidsequenz mit der GenBank/EMBL-Datenbank-Zugangsnummer Z21961 passen, wurden benutzt, um die cDNA der Disintegrin-Metalloprotease ADAM 10 aus dem Rind aus einer Rindernieren-cDNA-Bibliothek zu amplifizieren. Die cDNA von ADAM 10 wurde ohne das Original-Stop-Codon amplifiziert. Statt dessen wurde eine KpnI-Schnittstelle eingeführt und für die Fusion der ADAM 10-cDNA mit einer synthetischen DNA-Kassette, die für das Influenza-Hämagglutinin (HA)-Epitop-tag (YPYDVPDYA) codiert, benutzt. Der Vergleich der klonierten Nucleotidsequenz von Rinder-ADAM 10 wurde mit der publizierten Original-Sequenz [Howard et al. (1996) (Biochem. J. 317, Seiten 45 bis 50, GenBank/EMBL-Datenbank-Zugangsnummer Z21961] verglichen und zeigte die folgenden Austausche: C700T, C2122T und C2251T (die Nukleotidkoordinaten entsprechen der Sequenz mit der Zugangsnummer Z21961). Die Austausche verändern nicht die Aminosäurenabfolge.

Zur Expression der carboxyterminal HA-markierten Disintegrin-Metalloprotease ADAM 10 wurde der Expressionsvektor pcDNA3 (Invitrogen BV, Groningen, Niederlande) benutzt. Die stabile Transvektion von HEK-Zellen wurde nach der Calciumphosphat-Präzipitationstechnik durchgeführt. Zur Selektion transfizierter Zellen wurde 1 mg/ml Geneticin benutzt. Die Überexpression von ADAM 10 beschleunigte das Wachstum von transfizierten HEK-Zellen nicht.

Um die dominante negative Mutante der Disintegrin-Metalloprotease ADAM 10 aus dem Rind (mit DN bezeichnet, siehe Fig. 7, B) herzustellen, wurde eine Aminosäuresubstitution Alanin für Glutamat bei Position 384 (E384A) mittels PCR-Mutagenese durchgeführt (Die Nummerierung der Aminosäuren in der Angabe E384A bezieht sich auf die publizierte Sequenz der Disintegin Metalloprotase ADAM 10 des Rindes gemäß Howard et al. (1996) Biochem. J. 317, Seiten 45 bis 50, GenBank/EMBL-Datenbank-Zugangsnummer Z21961). Die amplifizierte cDNA der ADAM-10-Mutante wurde in pcDNA3 einkloniert und eine DNA-Kassette, die für ein FLAG-Epitop-tag (DYKDDDDK) kodiert, am 3'-Ende eingefügt. Die korrekte Sequenz aller PCR-Produkte wurde mit Didesoxynucleotid-Sequenzierung überprüft. HEK-Zellen wurden, wie vorstehend beschrieben, transfiziert und selektiert. Um die Expression der ADAM-10-Mutante zu überprüfen, wurde das Protein mit Western Blot Analyse unter Verwendung eines M2 Anti-FLAG-Antikörpers (Kodak) und dem ECL-Detektionssystems (Amersham Pharmacia Biotech) nachgewiesen.

### Beispiel 5

### Reverse Transkriptase - Polymerasekettenreaktion(PCR):

Gesamt-RNA von MDBK-Zellen (Madin Darby bovine kidney) und HEK-Zellen wurde unter Verwendung des RNeasy Kits (Qiagen GmbH, Hilden) hergestellt. Zur reversen Transkription der menschlichen und der Rinder-ADAM-10 mRNA wurde ein genau passendes Oligonucleotid für das 3'-Ende der mRNAs benutzt (5'-TGCACCGCATGAAAACATC-3'). Die PCR wurde durch Zugabe eines Vorwärtsprimers 5'-GAACAAGGTGAAGAATGTG-3' mit einer Sequenz durchgeführt, die in der menschlichen und in der Rinder-ADAM-10 cDNA identisch ist. Um die Herkunft der amplifizierten Fragmente als revers transkribierte mRNA sicherzustellen, wurde RNA vor Zugabe der Reversen Transkriptase in einem Kontrollversuch durch RNase A abgebaut.

### Beispiel 6

### Expression und Deglycosilierung der Disintegrin-Metalloprotease ADAM 10:

Stabil ADAM 10 exprimierende HEK-Zellen und Veraleichszellen wurden in Dulbecco's Modified Eagle's Medium (DMEM) supplementiert mit 10 % fötalem Kälberserum, Penicillin (100 Einheiten pro ml), Streptomycin (100 µg/ml) und 2 mM Glutamin auf Kulturschalen eines Durchmessers von 10 cm bis zur Konfluenz hochgezogen. Die Zellen wurden 20 Min. auf Eis in 1 ml Lysispuffer lysiert (20 mM Imidazol pH 6,8, 0,2 % Triton X-100, 100 mM KCl, 2 mM MgCl₂, 10 mM EGTA, 300 mM Saccharose, 1 mg/ml BSA) in Anwesenheit eines Inhibitors (complete^{™} Mini, Boehringer Mannheim). Die geklärten Extrakte wurden mit 6,6 µg/ml monoklonalem anti-HA-Antikörper (16B12) und Protein A-Sepharose bei 4°C über Nacht inkubiert. Für Deglycosilierungsexperimente wurden die Immunkomplexe in 100 µl 1 % SDS und 2 % β-Mercaptoethanol 10 min gekocht. Nach Zentrifugation wurde das Eluat mit 2.500 Einheiten PNGase F (New England Biolabs, Inc., Massachusetts. USA) in 50 mM Natriumphosphat pH 7,5, 1,5 % Nonidet-P40 und 0,25 % SDS 3 Stunden bei 37°C inkubiert. Die Proteine wurden wie bei Wessel und Flügge (1984) (Anal. Biochem. 138, Seiten 141 bis 143) präzipitiert, mit Hilfe eines 4 bis 12 %-igen NuPAGE-Gels (Nowex) getrennt und auf PVDF-Membranen geblottet. Die Membranen wurden mit polyklonalem anti-HA-Antikörper (Y-11) bei einer Verdünnung von 1:1.000 inkubiert, gefolgt von einem sekundären anti-Kaninchen-Meerrettichperoxidasegekoppelten Antikörper. Die Detektion erfolgte mit Hilfe des ECL-Systems.

### Beispiel 7

### Zelloberflächenbiotinylierungen:

Die Zellen wurden bis zur 90 %-igen Konfluenz herangezogen und mit PBS enthaltend 0,3 mg/ml Sulfo-NHS-LC-Biotin (Pierce, Rockford, Illinois, USA) 30 Min. bei 4 °C inkubiert. Überschüssiges Biotinylierungsreagenz wurde durch mehrmaliges Waschen mit TBS entfernt. Die Zellysate wurden mit monoklonalem anti-HA-Antikörper wie vorstehend beschrieben immunopräzipitiert. Die Immunkomplexe wurden von gewaschenen Protein-A- Beads mit 200 µl 100 mM Glycin pH 2,5 und 20 µM kompetierendes HA-Peptid (entsprechend den Aminosäuren 98 bis 108 des Influenza Hämaglutinin-Proteins) eluiert. Die Überstände wurden gesammelt und mit 5 µl 3 M Tris pH 8,9 neutralisiert und mit Streptavidin-Agarose-Beads (Pierce, Rockford, Illinois, USA) zwei Stunden bei 4°C inkubiert. Die Proben wurden mit Hilfe von 10 % SDS PAGE analysiert und auf PVDF-Membranen geblottet. Die Blots wurden wie vorstehend beschrieben analysiert.

### Beispiel 8

### Immunoblotanalyse:

8.10⁵ HEK und HEK-ADAM10-Zellen (HEK-Zellen, die ADAM 10 stabil exprimieren, Verfahren wie oben) wurden auf 5 cm Zellkulturschalen, die mit Poly-L-Lysin behandelt worden waren, ausgesät und fast bis zu Konfluenz hochgezogen. Das Medium wurde verworfen und die Zellen in Abwesenheit oder Anwesenheit der aufgeführten Verbindung 4 Stunden in Serum-freien DMEM mit 10 µg/ml Fettsäure-freiem BSA inkubiert. Das Medium wurde gesammelt und die Proteine mit 10 % (v/v) TCA präzipitiert. Die Proben wurden bei 14.000 UpM zentrifugiert und die Pellets zweimal mit 500 µl eiskaltem Aceton gewaschen. Die Proteine wurden mit 7,5 % SDS-PAGE getrennt und auf PVDF-Membranen geblottet. Die Membranen wurden mit monoklonalem Antikörper 6E10, der die aminoterminale Sequenz von Aβ erkennt, bei einer Verdünnung von 1:2.000 gefolgt von einem sekundären [³⁵S]-markierten anti-Maus IgG-Antikörper inkubiert. Die radioaktiven Banden, die APPsα entsprachen, wurden mit Hilfe des Bio-Imaging-Analysers-BAS-1800 (Fuji) quantifiziert. Der Proteingehalt jeder Zellkulturplatte wurde nach Dialyse mit dem Mikro-BCA Proteinassay (Pierce, Rockford, Illinois, USA) bestimmt und die Meßwerte der radioaktiven Banden auf die Proteinmenge normiert.

### Beispiel 9

### Metabolische Markierung und Immunopräzipitation:

1·10 ⁶ HEK-Zellen und HEK-ADAM10-Zellen wurden wie oben beschrieben auf 10 cm Zellkulturplatten hochgezogen. Die fast konfluenten Zellkulturen wurden in Serum-freien und Cystein- und Methionin-freiem DMEM 1 Stunde inkubiert, und dann in demselben Medium mit 0,2 mCi/ml Tran[³⁵S]-label^{™} (ICN) 5 Stunden markiert. Die Immunopräzipitationen wurden ausgeführt, wie in Haass et al. (1991) (J. Neurosci. 11, Seiten 3.783 bis 3.793) und Haass et al. (1992) (Nature 359, Seiten 322 bis 335) beschrieben. APPSα wurde mit dem α-Sekretasespezifischen polyklonalen Antikörper 1736 und das 10 kDa-Fragment (p10) mit dem polyklonalen Antikörrper C7, der den carboxyterminalen Teil von APP erkennt, immunpräzipitiert. Die Radioaktivität wurde mit Hilfe des Bio-Imaging-Analysers BAS-.1800 (Fuji) detektiert.

### Beispiel 10

### Spezifität der gereinigten Disintegrin-Metalloprotease ADAM 10 für verschiedene Peptidsubstrate.

Als Peptidsubstrat, das die α-Sekretase-Schnittstelle umfaßt, wurde die Octapeptid-Aminosäuresequenz 11 bis 28 von Aβ gewählt (die Numerierung bezieht sich auf den Aminoterminus des β-Amyloid-Peptids (Esch et al. (1990) Science 248, Seiten 1122 bis 1124 und Haass et al. (1992) Nature 359, Seiten 322 bis 325). Seine carboxyterminale Aminosäure entspricht der ersten extrazellulären Aminosäure von APP. Nach 30 min Inkubation von Aβ(11-28) mit ADAM 10 zeigte die HPLC-Analyse eine Spaltungsausbeute von 28 % bezogen auf das Startpeptid und unter Bildung von zwei Proteolyseprodukten (siehe Tabelle 2).

Die ESI-massenspektrometrische Analyse identifizierte die Proteolyseprodukte als das aminoterminale Hexapeptid ([M+H] ⁺-Ion mit m/z von 777,5) und als das carboxyterminale Dodecapeptidamid ([M+H]⁺-Ion mit m/z von 2.082,8) des ursprünglichen Octadecapeptidamids. Folglich spaltet die Disintegrin-Metalloprotease ADAM-10 proteolytisch zwischen Lys 16 und Leu 17 (Nummerierung beginnend mit dem Aminoterminus von Aβ), wie es für ein Enzym mit α-Sekretaseaktivität zu erwarten ist. Nach 6-stündiger Inkubation waren 69 % des Aβ(11-28) vorwiegend an dieser Seite gespalten, seltener erfolgte eine Spaltung hinter Leu 17 und Val 18 (Fig. 3 A; Tabelle 2).

Anschließend wurde überprüft, ob die Spaltungshäufigkeit wie für die Spaltung von APP durch die α-Sekretase beschrieben worden ist (siehe Sisodia (1992) Proc. Natl. Acad. Sci USA 89, Seiten 6075 bis 6079) von der Konformation des Substrats abhängt. Zu diesem Zweck wurde Alanin 21 in Aβ(11-28) durch Lysin ersetzt. Diese Position entspricht einer natürlich vorkommenden Ala→Gly-Mutation bei Position 692 von APP₇₇₀ (Hendricks et al. (1992) Nat. Genet. 1, Seiten 218 bis 221), die in Patienten mit zerebraler Hämorrhagie in Folge von Amyloidangiopathie identifiziert wurde (Haass et al (1994) J. Biol. Chem. 269, Seiten 17741 bis 17748; Capell et al. (1996) Amyloid 3, Seiten 150 bis 155).

Cirkulardichroismus-Messungen (CD) des Octadecapeptidamids Aβ(11-28) in 0,5 % SDS zeigten ein Spektrum, das charakteristisch für die α-helicale Konformation ist, während eine Zufallsknäul-Konformation im Falle des Peptids mit dem Austausch Ala→Gly in Position 21 von Aβ(11-28) (siehe Fig. 3 B) beobachtet wurde. Das letzte Peptid wurde durch die Disintegrin-Metalloprotease ADAM 10 weniger effektiv als das Wildtyp-Peptid gespalten: Nach 30 Min. wurde nur 14 % zwischen Lys 16 und Leu 17 gespalten, verglichen mit 28 % im Falle von Aβ(11-28) (siehe Tabelle 2). Ein Vergieichsbeispiel zeigt, daß ADAM 10 nicht die Peptidsubstrate spaltet, die eine Schnittstelle für das sog. *Ectodomain Shedding,* das Abwerfen einer Außendomäne, des Angiotensin umwandelnden Enzyms (ACE) (Ehlers et al (1996) Biochemistry 35, Seiten 9549 bis 9559) oder des Interleukin 6-Rezeptors (Müllberg et al. (1994) J. Immunol. 152, Seiten 4958 bis 4968), besitzen. Allerdings spaltet ADAM-10 offensichtlich ein von pro-TNF-α abgeleitetes Peptidsubstrat, wie neulich berichtet wurde (Lunn et al. (1997) FEBS Lett. 400, Seiten 333 bis 335; Rosendahl et al (1997) J. Biol. Chem. 272, Seiten 24588 bis 24593). Einige Metalloproteinase-Inhibitoren wurden auf ihre Fähigkeit, die proteolytische Spaltung von Aβ(11-28) durch gereinigtes ADAM 10 zu inhibieren, getestet. Der auf einer Hydroxamsäure basierende Inhibitor BB-3103 (Middelhoven et al. (1997) FEBS Lett. 414, Seiten 14 - 18) inhibierte bei einer Konzentration von 100 µM vollständig die proteolytische Spaltungsaktivität. Die Disintearin-Metalloprotease ADAM 10 wurde auch durch 1 mM DTT vollständig inhibiert, was darauf schließen läßt, daß eine Thiol-Gruppe oder Disulfidbindungen wichtig für die a-Sekretaseaktivität sind. Ferner inhibierte ADAM-10 1 mM 1,10-Phenanthrolin.

### Beispiel 11

### Prozessierung und Lokalisation der Metalloprotease ADAM 10 in HEK-Zellen

Um die Wirkung der Metalloprotease ADAM 10 auf die proteolytische Spaltung von APP in einem Zellsystem zu untersuchen, wurden HEK 293-Zellen eingesetzt. Die cDNA der Metalloprotease ADAM 10 wurde aus einer Rindernieren cDNA-Bibliothek kloniert an ihrem 3'- mit einer DNA-Sequenz, die für das HA-Antigen kodiert, versehen und in den Expressionsvektor pcDNA3 (Invitrogen BV, Groningen, Niederlande) kloniert. Nach der Transfektion von HEK-Zellen und HEK-Zellen, die APP₆₉₅ stabil exprimieren [HEK APP₆₉₅, Haass et al. (1992), Nature 359, Seiten 322 - 325], mit Nukleinsäure, kodierend für die Metalloprotease ADAM 10, wurden Klone auf die Expression und Prozessierung der Metalloprotease hin untersucht. Zu diesem Zweck wurden Zellysate mit dem monoklonalen anti-HA-Antikörper (16B12) präzipitiert mit SDS-PAGE analysiert und einem Western Blot unterzogen. Immuno-Anfärbungen mit einem anti-HApolyklonalen Antikörper zeigten 2 immunoreaktive Spezies einer apparenten Größe von 90 kDa und 64 kDa (Fig. 4 A, Spuren 2 - 5). Die 64 kDa-Variante wird aus der 90 kDa-Variante durch Abspaltung der Pro-Domäne (194 Aminosäuren) gebildet. Wahrscheinlich geschieht dies durch eine Furin-ähnliche Pro-Proteinkonvertase, die die Disintegrin-Metalloproteasen bei dem Sequenzmotiv RXKR in einem späten Golgi-Kompartiment spaltet (Lum et al. (1998) J. Biol. Chem. 273, Seiten 26236 - 26247). Die berechnete Molekularmasse der Metalloprotease ADAM 10 nach der proteolytischen Abspaltung der Prodomäne ist 61 kDa. Um zu untersuchen, ob die Differenz zwischen apparenten und berechneten Molekulargewicht auf die Glycosilierung an den putativen N-Glycosilierungsstellen der Disintegrin-Metalloprotease ADAM 10 zurückzuführen ist (Howard et al. 1996) Biochem. J. 317, Seiten 45 - 50), wurden die Glycoproteine des Zellklons mit dem höchsten Expressionsspiegel an Disintegrin-Metalloprotease ADAM 10 (HEK-ADAM 10) mit N-Glycosidase F behandelt. Diese Behandlung führte zu einer Reduktion der Molekularmasse auf die erwarteten Werte von etwa 86 kDa für das Vorläuferprotein und von 61 kDa für die prozessierte Variante der Disintearin-Metalloprotease ADAM 10, der die Prodomäne fehlt (Fig. 4 A, Spur 3).

Die Lokalisation der Disintegrin-Metalloprotease ADAM 10 in transfizierten HEK-Zellen wurde durch Zelloberflächen-Biotinylierung und konfokale Mikroskopie untersucht. Zur Biotinylierung wurden die Zellen auf 4°C gekühlt und 30 Minuten mit Sulfo-NHS-LC-Biotin, einem membranimpermeabelen Biotinylierungsreagenz, inkubiert. Die Immunopräzipitation wurde unter Verwendung eines monoklonalen Anti-HA-Antikörpers durchgeführt. 4/5 der gesammelten Immunopräzipitate wurden mit immobilisiertem Streptavidin inkubiert, um die Proteasemoleküle an der Plasmamembran zu isolieren. Fig. 4 B zeigt die Anwe-senheit von sowohl der prozessierten etwa 64 kDa großen Variante der Disintegrin-Metalloprotease ADAM 10 als auch ihres 90 kDa großen Vorläufers auf der Zelloberfläche (Spur A). 1/5 des Immunopräzipitates, das nicht mit Streptavidin inkubiert worden war, zeigte vornehmlich die nicht-prozessierte 90 kDa große Variante, und eine nur schwache Bande wies auf die proteolytisch aktivierte Disintegrin-Metalloprotease ADAM 10 hin (Spur B). Diese Ergebnisse zeigen, daß die proteolytisch aktivierte Form der Metalloprotease ADAM 10 vornehmlich an der Zelloberfläche lokalisiert ist, wo sie in der Lage ist APP proteolytisch zu spalten. Der überwiegende Teil der Disintegrin-Metalloprotease ADAM 10 liegt intrazellular als Proenzym vor. Eine Analyse der Lokalisation der Disintegrin-Metalloprotease ADAM 10 mit Hilfe von konfokaler Immunofluoreszenzmikroskopie an permiabilisierten HEK-ADAM 10 Zellen zeigte, daß das Anfärbemuster größtenteils mit dem des Golgi-assoziierten Markers 58k überlappt (Ergebnisse nicht gezeigt).

### Beispiel 12

### Wirkung der Disintegrin-Metalloprotease ADAM 10 auf die a-Sekretase Spaltung von APP in HEK-Zellen.

Die α-Sekretaseaktivität in HEK-ADAM 10 Zellen wurde mit der Aktivität, die in untransfizierten HEK-Zellen gefunden wurde, verglichen. Die Freisetzung von APPs in das Medium wurde mit zwei bindungsortsspezifischen Antikörpern (1736 und 6E10) bestimmt. Beide Antikörper erkennen die aminoterminale Sequenz von Aβ und detektieren daher nur APPsα aber nicht APPsβ. Immunoblotexperimente und Immunopräzipitation nach metabolischer [³⁵S]-Markierung wurden durchgeführt, um die α-Sekretaseaktivität zu quantifizieren (Fig. 5 und Fig. 6). Die quantitative Analyse der lmmunoblotexperimente mit dem monoklonalem Antikörper 6E10 zeigte, daß HEK-Zellen, die stabil einen hohen Spiegel der Disintegrin-Metalloprotease ADAM 10 exprimieren, ungefähr die vierfache Menge APPsα ins Medium abgeben als die untransfizierten HEK-Zellen (Fig. 5, Spuren 1-4). Eine erhöhte α-Sekretaseaktivität wurde auch in einem HEK-APP₆₉₅-Zellkion, der stabil die Disintegrin-Metalloprotease ADAM 10 exprimiert, festgestellt. Der relative Anstieg von APPs₇₅₁α, welches von endogenem APP abstammt und von APPs₆₉₅α war nur zweifach (Fig. 5A, Spuren 7 und 8) infolge des geringeren Expressionsspiegels der Disintegrin-Metalloprotease ADAM 10 (Fig. 4, Spur 5) und infolge des höheren Substrat-Enzymverhältnisses.

Neulich wurde gezeigt, daß Zink-Metalloproteaseinhibitoren auf der Basis von Hydroxamsäure das *Ectodomain Shedding* verschiedener Membranproteine einschließlich der α-Sekretase-Spaltung von APP unterdrücken. Ein Effekt auf die Freisetzung von APPsβ wurde nicht gefunden (Parvathy et al. (1998) Biochemistry 37, Seiten 1680-1685). Deswegen wurde der Effekt des Hydroxamsäureinhibitors BB-3103 auf HEK-Zellen und auf HEK-Zellen, die die Disintegrin-Metalloprotease ADAM 10 überexprimieren, untersucht. BB-3103 verminderte die Freisetzung von APPsα in HEK-Zellen um etwa 40 % (Fig. 5, Spuren 1-3) und in HEK-ADAM 10-Zellen um etwa 70 % (Fig. 5, Spuren 4-6). Die Stimulation der Proteinkinase C durch Phorbolester verstärkt deutlich die Freisetzung von APPsα und inhibiert die Sekretion von Aβ. Um die Wirkung der Proteinkinase C auf die Stimulierung der Disintegrin-Metalloprotease ADAM 10-Aktivität zu untersuchen, wurden HEK- und HEK-ADAM 10-Zellen mit 1 µm PMA behandelt. Immunoblotexperimente zeigten, daß PMA die APPsα-Freisetzung von HEK-Zellen etwa um das 6-fache erhöht (Fig. 5, Spuren 1 und 2). Die verstärkte α-Sekretaseaktivität von HEK-ADAM 10-Zellen wurde um den Faktor 2,5 vergrößert (Fig. 5, Spuren 4 und 5).

ADAM 10 besitzt in Aminosäureposition 719 ein Threonin, welches als Substrat für eine Proteinkinase C-abhängige Phosphorylierung dienen kann. Eine Verstärkung der α-Sekretaseaktivität von ADAM 10 durch den Proteinkinase C-Aktivator PMA erfolgt offensichtlich über diesen Mechanismus.

Ähnliche Ergebnisse wurden in Immunopräzipitationsexperimenten mit dem polyklonalen Antikörper 1736 erhalten. Die Expression der Disintegrin-Metalloprotease ADAM 10 in HEK-Zellen verstärkte signifikant die Sekretion von APPsα in das Medium (Fig. 6A). Um zu bestimmen, ob die Expression der Disimegrin-Metalloprotease ADAM 10 auch den Spiegel von p 10 erhöht, wurden Zellysate von HEK- und HEK-ADAM 10-Zellen nach metabolischer Markierung mit dem polyklonalen Antikörper C7, der den carboxyterminalen Teil von APP erkennt, immunopräzipitiert. Wie in Fig. 6B und 6C gezeigt, wird die Expression von Holo-APP (APP der vollen Länge) nicht verändert, während das Signal von p10 in HEK-ADAM 10-Zellen signifikant stärker ist als in den Veraleichszellen. Dies zeigt, daß die Disintearin-Metalloprotease ADAM 10 α-Sekretaseaktivität besitzt.

### Beispiel 13

### Wirkung der dominant negativen Disintegrin-Metalloprotease ADAM 10-Mutante auf die α-Sekretaseaktivität:

Um festzustellen, ob das menschliche Homologe der Disintegrin-Metalloprotease ADAM 10 in HEK-Zellen exprimiert wird und daher für die endogene α-Sekretaseakrtivität verantwortlich ist, wurden Reverse Transkriptase Polymerasekettenreaktionsanalysen unter Verwendung von Gesamt-RNA aus diesen Zellen und Oligonucleotiden, die spezifisch für ADAM 10 sind, durchgeführt. Wie in Fig. 7A gezeigt, exprimieren HEK-Zellen detektierbare Mengen an mRNA, die für die humane Variante der Disintegrin-Metalloprotease ADAM 10 codiert, die 97 % Identität auf Aminosäureebene mit der Disintegrin-Metalloprotease ADAM 10 aus dem Rind zeigt. In einem Vergleichsexperiment wurde die Disintegrin-Metalloprotease ADAM 10-mRNA in Gesamt-RNA von MDBK-Zellen nachgewiesen, von denen bekannt ist, daß sie die Disintegrin-Metalloprotease ADAM 10 exprimieren (Howard et al. (1996) Biochem. J. 317, Seiten 45-50).

Um die endogene α-Sekretaseaktivität in HEK-Zellen zu inhibieren, wurde die Punktmutation E384A in die Zink-Bindungsstelle der Rinder-Disintegrin-Metalloprotease ADAM 10 eingeführt. Eine Mutante des KUZ-Proteins mit einer Mutation an derselben Stelle verhielt sich dominant negativ in *Drosophila melanogaster* (Pan und Rubin (1997) Cell 90, Seiten 271-280). HEK-Zellen, die die Mutante ADAM 10 E384A (HEK/DN) exprimieren, zeigten eine deutlich verminderte Sekretion von APPs. Der inhibitorische Effekt war am deutlichsten in PMA-behandelten Zellen: Nur 25 % der enzymatischen Aktivität wurde festgestellt (Fig. 7B, Spuren 2 und 4; Fig. 7C). Folglich führte diese Punktmutation zu einer dominant negativen Form der Protease und zu einer deutlich verminderten konstitutiven und stimulierbaren α-Sekretaseaktivität.

Zusammenfassend läßt sich also sagen, daß die Disintegrin-Metalloprotease ADAM 10 α-Sekretaseaktivität besitzt und in den basalen und stimulierten Prozeß des *Ectodomain Sheddings* von APP involviert ist. Diese Folgerung ergibt sich im wesentlichen aus folgenden experimentellen Ergebnissen:
- Die Überexpression der Disintegrin-Metalloprotease ADAM 10 in HEK-Zellen führt zu einem Anstieg von APPsα und p10 um ein vielfaches. Der Anstieg in der Proteolyseaktivität (Shedding-Aktivität) korreliert mit dem Expressionslevel der Disintegrin-Metalloprotease ADAM 10. Die verstärkte α-Sekretaseaktivität kann durch Stimulation der Proteinkinase C mit Phorbolestern erhöht werden, was ein charakteristisches Merkmal der α-Sekretase ist (HUNG et al. (1993) J. Biol. Chem. 268, Seiten 22959-22962).
- Zelloberflächen-Biotinylierungsexperimente zeigen, daß die proteolytisch aktivierte Form der Disintegrin-Metalloprotease ADAM 10 im wesentlichen in der Plasmamembranen lokalisiert ist.
- Die endogene basale und Phorbolester stimulierte α-Sekretaseaktivität in HEK-Zellen kann durch eine dominant negative Form der Disintegrin Metalloprotease ADAM 10 deutlich inhibiert werden.
- Das Inhibitorspektrum sowohl des isolierten als auch des überexprimierten Enzyms stimmt mit den Berichten über die Hemmung der α-Sekretaseaktivität überein.

Auf die Veröffentlichung Lammich *et al.* Proc. Natl. Acad. Sci USA (1999) 96, 3922-7 wird vollinhaltlich verwiesen.

**Tabelle 2**

| **Protein** | **Sequenz** | **% Spaltung durch Rinder-ADAM 10 nach** | |
|---|---|---|---|
| | | **30 min** | **6 h** |
| Pro-TNF | PLAQA↓VRSSSSRTPSD-NH₂ | 49 | 100 |
| β-Amyloidvorläuferprotein | EVHHQK↓LVFFAEDVGSNK-NH₂ EVHHQK↓LVFFGEDVGSNK-NH₂ | 28 | 69 |
| H-6-Rezeptor | ANATFLPVQ↓DSSSV-NH₂ | 14 | 54 |
| Angiotensin umwandelndes Enzym | TPNSAR↓SEGPLPDSGR-NH₂ | 0 | 0 |

### SEQUENZPROTOKOLL

ALLGEMEINE ANGABEN:
   - ANMELDER:: NAME: Professor Falk Fahrenholz
   STRASSE: Becherweg 30
   BUNDESLAND: Rheinland-Pfalz
   LAND: Deutschland
   POSTLEITZAHL: 55099
   TELEFON: 06131395833
   TELEFAX: 06131395348
   - BEZECHNUNG DER ERFINDUNG:: Zellen, die ein Amyloidvorläuferprotein und eine alpha- Sekretase coexprimieren, ein Testverfahren zur Identifikation alpha-Sekretase-aktiver Substanzen sowie eines zur Identifikation weiterer Sekretasen, ein Testverfahren zur Bestimmung der Anfälligkeit gegenüber *Morbus Alzheimer* und Verwendung von Nukleinsäuren, die für eine alpha-Sekretase codieren, für die Gentherapie
ANZAHL DER SEQUENZEN: 2
COMPUTERLESBARE FASSUNG:
   DATENTRÄGER: Diskette
   COMPUTER: IPM-kompatibel
   BETRIEBSYSTEM: WINDOWS NT
   SOFTWARE: WORD97
ANGABEN ZU SEQ ID-NO:1:
SEQUENZCHARKTERISTIKA
   LÄNGE: 627 Aminosäuren
   ART: Aminosäure
   TOPOLOGIE: linear
ART DES MOLEKÜLS: Protein
SEQUENZBESCHREIBUNG: SEQ ID-No:1: 
Met Leu Leu Leu Leu Leu Leu Leu Gly Leu Arg Leu Gln Leu Ser
                5                   10                  15
Leu Gly Ile Ile Pro Val Glu Glu Glu Asn Pro Asp Phe Trp Asn
                20                  25                  30
Arg Glu Ala Ala Glu Ala Leu Gly Ala Ala Lys Lys Leu Gln Pro
                35                  40                  45
Ala Gln Thr Ala Ala Lys Asn Leu Ile Ile Phe Leu Gly Asp Gly
                50                  55                  60
Met Gly Val Ser Thr Val Thr Ala Ala Arg Ile Leu Lys Gly Gln
                65                  70                  75
Lys Lys Asp Lys Leu Gly Pro Glu Ile Pro Leu Ala Met Asp Arg
                80                  85                  90
Phe Pro Tyr Val Ala Leu Ser Lys Thr Tyr Asn Val Asp Lys His
                95                  100                 105
Val Pro Asp Ser Gly Ala Thr Ala Thr Ala Tyr Leu Cys Gly Val
                110                 115                 120
Lys Gly Asn Phe Gln Thr Ile Gly Leu Ser Ala Ala Ala Arg Phe
                125                 130                 135
Asn Gln Cys Asn Thr Thr Arg Gly Asn Glu Val Ile Ser Val Met
                140                 145                 150
Asn Arg Ala Lys Lys Ala Gly Lys Ser Val Gly Val Val Thr Thr
                155                 160                 165
Thr Arg Val Gln His Ala Ser Pro Ala Gly Thr Tyr Ala His Thr
                170                 175                 180
Val Asn Arg Asn Trp Tyr Ser Asp Ala Asp Val Pro Ala Ser Ala
                185                 190                 195
Arg Gln Glu Gly Cys Gln Asp Ile Ala Thr Gln Leu Ile Ser Asn
                200                 205                 210
Met Asp Ile Asp Val Ile Leu Gly Gly Gly Arg Lys Tyr Met Phe
                215                 220                 225
Arg Met Gly Thr Pro Asp Pro Glu Tyr Pro Asp Asp Tyr Ser Gln
                230                 235                 240
Gly Gly Thr Arg Leu Asp Gly Lys Asn Leu Val Gln Glu Trp Leu
                245                 250                 255
Ala Lys Arg Gln Gly Ala Arg Tyr Val Trp Asn Arg Thr Glu Leu
                260                 265                 270
Met Gln Ala Ser Leu Asp Pro Ser Val Thr His Leu Met Gly Leu
                275                 280                 285
Phe Glu Pro Gly Asp Met Lys Tyr Glu Ile His Arg Asp Ser Thr
                290                 295                 300
Leu Asp Pro Ser Leu Met Glu Met Thr Glu Ala Ala Leu Arg Leu
                305                 310                 315
Leu Ser Arg Asn Pro Arg Gly Phe Phe Leu Phe Val Glu Gly Gly
                320                 325                 330
Arg Ile Asp His Gly His His Glu Ser Arg Ala Tyr Arg Ala Leu
                335                 340                 345
Thr Glu Thr Ile Met Phe Asp Asp Ala Ile Glu Arg Ala Gly Gln
                350                 355                 360
Leu Thr Ser Glu Glu Asp Thr Leu Ser Leu Val Thr Ala Asp His
                365                 370                 375
Ser His Val Phe Ser Phe Gly Gly Tyr Pro Leu Arg Gly Ser Ser
                380                 385                 390
 
Ile Phe Gly Leu Ala Pro Gly Lys Ala Arg Asp Arg Lys Ala Tyr
                395                 400                 405
Thr Val Leu Leu Tyr Gly Asn Gly Pro Gly Tyr Val Leu Lys Asp
                410                 415                 420
Gly Ala Arg Pro Asp Val Thr Glu Ser Glu Ser Gly Ser Pro Glu
                425                 430                 435
Tyr Arg Gln Gln Ser Ala Val Pro Leu Asp Glu Glu Thr His Ala
                440                 445                 450
Gly Glu Asp Val Ala Val Phe Ala Arg Gly Pro Gln Ala His Leu
                455                 460                 465
Val His Gly Val Gln Glu Gln Thr Phe Ile Ala His Val Met Ala
                470                 475                 480
Phe Ala Ala Cys Leu Glu Pro Tyr Thr Ala Cys Asp Leu Ala Pro
                485                 490                 495
Pro Ala Gly Thr Thr Asp Ala Ala His Pro Gly Val Tyr Thr Arg
                500                 505                 510
Pro Gly Ser Gly Leu Thr Asn Ile Lys Thr Glu Glu Ile Ser Glu
                515                 520                 525
Val Lys Met Asp Ala Glu Phe Arg His Asp Ser Gly Tyr Glu Val
                530                 535                 540
His His Gln Lys Leu Val Phe Phe Ala Glu Asp Val Gly Ser Asn
                545                 550                 555
Lys Gly Ala Ile Ile Gly Leu Met Val Gly Gly Val Val Ile Ala
                560                 565                 570
Thr Val Ile Val Ile Thr Leu Val Met Leu Lys Lys Lys Gln Tyr
                575                 580                 585
Thr Ser Ile His His Gly Val Val Glu Val Asp Ala Ala Val Thr
                590                 595                 600
Pro Glu Glu Arg His Leu Ser Lys Met Gln Gln Asn Gly Tyr Glu
                605                 610                 615
Asn Pro Thr Tyr Lys Phe Phe Glu Gln Met Gln Asn
                620                 625
ANGABEN ZU SEQ ID-NO:2:
SEQUENZCHARKTERISTIKA:
   LANGE: 1884 Basenpaare
   ART: Nucleinsäure
   STRANGFORM: Einzelstrang
   TOPOLOGIE: linear
ART DES MOLEKÜLS: c-DNA zu m-RNA
SEQUENZBESCHREIBUNG: SEQ ID-No:2: 
ATGCTGCTGC TGCTGCTGCT GCTGGGCCTG AGGCTACAGC TCTCCCTGGG CATCATCCCA 60
GTTGAGGAGG AGAACCCGGA CTTCTGGAAC CGCGAGGCAG CCGAGGCCCT GGGTGCCGCC 120
AAGAAGCTGC AGCCTGCACA GACAGCCGCC AAGAACCTCA TCATCTTCCT GGGCGATGGG 180
ATGGGGGTGT CTACGGTGAC AGCTGCCAGG ATCCTAAAAG GGCAGAAGAA GGACAAACTG 240
GGGCCTGAGA TACCCCTGGC CATGGACCGC TTCCCATATG TGGCTCTGTC CAAGACATAC 300
 
AATGTAGACA AACATGTGCC AGACAGTGGA GCCACAGCCA CGGCCTACCT GTGCGGGGTC 360
AAGGGCAACT TCCAGACCAT TGGCTTGAGT GCAGCCGCCC GCTTTAACCA GTGCAACACG 420
ACACGCGGCA ACGAGGTCAT CTCCGTGATG AATCGGGCCA AGAAAGCAGG GAAGTCAGTG 480
GGAGTGGTAA CCACCACACG AGTGCAGCAC GCCTCGCCAG CCGGCACCTA CGCCCACACG 540
GTGAACCGCA ACTGGTACTC GGACGCCGAC GTGCCTGCCT CGGCCCGCCA GGAGGGGTGC 600
CAGGACATCG CTACGCAGCT CATCTCCAAC ATGGACATTG ACGTGATCCT AGGTGGAGGC 660
CGAAAGTACA TGTTTCGCAT GGGAACCCCA GACCCTGAGT ACCCAGATGA CTACAGCCAA 720
GGTGGGACCA GGCTGGACGG GAAGAATCTG GTGCAGGAAT GGCTGGCGAA GCGCCAGGGT 780
GCCCGGTATG TGTGGAACCG CACTGAGCTC ATGCAGGCTT CCCTGGACCC GTCTGTGACC 840
CATCTCATGG GTCTCTTTGA GCCTGGAGAC ATGAAATACG AGATCCACCG AGACTCCACA 900
CTGGACCCCT CCCTGATGGA GATGACAGAG GCTGCCCTGC GCCTGCTGAG CAGGAACCCC 960
CGCGGCTTCT TCCTCTTCGT GGAGGGTGGT CGCATCGACC ATGGTCATCA TGAAAGCAGG 1000
GCTTACCGGG CACTGACTGA GACGATCATG TTCGACGACG CCATTGAGAG GGCGGGCCAG 1060
CTCACCAGCG AGGAGGACAC GCTGAGCCTC GTCACTGCCG ACCACTCCCA CGTCTTCTCC 1120
TTCGGAGGCT ACCCCCTGCG AGGGAGCTCC ATCTTCGGGC TGGCCCCTGG CAAGGCCCGG 1180
GACAGGAAGG CCTACACGGT CCTCCTATAC GGAAACGGTC CAGGCTATGT GCTCAAGGAC 1240
GGCGCCCGGC CGGATGTTAC CGAGAGCGAG AGCGGGAGTC CGGAGTATCG GCAGCAGTCA 1300
GCAGTGCCCC TGGACGAAGA GACCCACGCA GGCGAGGACG TGGCGGTGTT CGCGCGCGGC 1360
CCGCAGGCGC ACCTGGTTCA CGGCGTGCAG GAGCAGACCT TCATAGCGCA CGTCATGGCC 1420
TTCGCCGCCT GCCTGGAGCC CTACACCGCC TGCGACCTGG CGCCCCCCGC CGGCACCACC 1480
GACGCCGCGC ACCCGGGGGT GTACACTCGA CCAGGTTCTG GGTTGACAAA TATCAAGACG 1540
GAGGAGATCT CTGAAGTGAA GATGGATGCA GAATTCCGAC ATGACTCAGG ATATGAAGTT 1600
CATCATCAAA AATTGGTGTT CTTTGCAGAA GATGTGGGTT CAAACAAAGG TGCAATCATT 1660
GGACTCATGG TGGGCGGTGT TGTCATAGCG ACAGTGATCG TCATCACCTT GGTGATGCTG 1720
AAGAAGAAAC AGTACACATC CATTCATCAT GGTGTGGTGG AGGTTGACGC CGCTGTCACC 1780
CCAGAGGAGC GCCACCTGTC CAAGATGCAG CAGAACGGCT ACGAAAATCC AACCTACAAG 1860
TTCTTTGAGC AGATGCAGAA CTAG
 **CLUSTAL W (1.82) multiple sequence alignment**
   Die ADAM10-Proteine aus Mensch, Maus und Rind sind nahezu identisch.
   Menschliches ADAM10 ist zu 96% identisch mit Rinder- und mit Maus-ADAM10.

ADAM10_human MVLLRVLILLLSWAAGMGGQYGNPLNKYIRHYEGLSYNVDSLHQKHQRAKRAVSHEDQFL 60
ADAM10_bovine MVLLRVLILLLSWVAGLGGQYGNPLNKYIRHYEGLSYDVDSLHQKHQRAKRAVSHEDQFL 60
ADAM10_Maus MVLPTVLILLLSWAAGLGGQYGNPLNKYIRHYEGLSYNVDSLHQKHQRAKRAVSHEDQFL 60
*** ********.**:*******************:**********************

ADAM10_human RLDFHAHGRHFNLRMKRDTSLFSDEFKVETSNKVLDYDTSHIYTGHIYGEEGSFSHGSVI 120
ADAM10_bovine RLDFHAHGRHFNLRMKRDTSLFSEEFRVETSNAVLDYDTSHIYTGHIYGEEGSFSHGSVI 120
ADAM10_Maus LLDFHAHGRQFNLRMKRDTSLFSDEFKVETSNKVLDYDTSHIYTGHIYGEEGSFSHGSVI 120
********:*************:**:***** ***************************

ADAM10_human DGRFEGFIQTRGGTFYVEPAERYIKDRTLPFHSVIYHEDDINYPHKYGPQGGCADHSVFE 180
ADAM10_bovine DGRFEGFIQTHGGTFYVEPAERYIKDRTLPFHSVIYHEDDIKYPHKYGPQGGCADHSVFE 180
ADAM10_Maus DGRFEGFIKTRGGTFYIEPAERYIKDRILPFHSVIYHEDDINYPHKYGPQGGCADHSVFE 180
********:*:*****:********** *************:******************

ADAM10_human RMRKYQMTGVEEVTQIPQEEHAAN-GPELLRKKRTTSAEKNTCQLYIQTDHLFFKYYGTR 239
ADAM10_bovine RMRKYQMTGVEEVTQTPQEKHAIN-GPELLRKKRTTVAEKNTCQLYIQTDHLFFKYYGTR 239
ADAM10_Maus RMRKYQMTGVEEGARAHPEKHAASSGPELLRKKRTTLAERNTCQLYIQTDHLFFKYYGTR 240
************ :: *:** . *********** **:*********************

ADAM10_human EAVIAQISSHVKAIDTIYQTTDFSGIRNISFMVKRIRINTTADEKDPTNPFRFPNIGVEK 299
ADAM10_bovine EAVIAQISSHVKAIDTIYQTTDFSGIRNISFMVKRIRINTTADEKDPTNPFRFPNIGVEK 299
ADAM10_Maus EAVIAQISSHVKAIDTIYQTTDFSGIRNISFMVKRIRINTTSDEKDPTNPFRFPNIGVEK 300
*****************************************:******************

ADAM10_human FLELNSEQNHDDYCLAYVFTDRDFDDGVLGLAWVGAPSGSSGGICEKSKLYSDGKKKSLN 359
ADAM10_bovine FLELNSEQNHDDYCLAYVFTDRDFDDGVLGLAWVGAPSGSSGGICEKSKLYSDGKKKSLN 359
ADAM10_Maus FLELNSEQNHDDYCLAYVFTDRDFDDGVLGLAWVGAPSGSSGGICEKSKLYSDGKKKSLN 360
************************************************************

ADAM10_human TGIITVQNYGSHVPPKVSHITFAHEVGHNFGSPHDSGTECTPGESKNLGQKENGNYIMYA 419
ADAM10_bovine TGIITVQNYGSHVPPKVSHITFAHEVGHNFGSPHDSGTECTPGESKNLGQKENGNYIMYA 419
ADAM10_Maus TGIITVQNYGSHVPPKVSHITFAHEVGHNFGSPHDSGTECTPGESKNLGQKENGNYIMYA 420
************************************************************

ADAM10_human RATSGDKLNNNKFSLCSIRNISQVLEKKRNNCFVESGQPICGNGMVEQGEECDCGYSDQC 479
ADAM10_bovine RATSGDKLNNNKFSLCSIRNISQVLEKKRNNCFVESGQPICGNGMVEQGEECDCGYSDQC 479
ADAM10_Maus RATSGDKLNNNKFSLCSIRNISQVLEKKRNNCFVESGQPICGNGMVEQGEECDCGYSDQC 480
************************************************************

ADAM10_human KDECCFDANQPEGRKCKLKPGKQCSPSQGPCCTAQCAFKSKSEKCRDDSDCAREGICNGF 539
ADAM10_bovine KDECCYDANQPEGKKCKLKPGKQCSPSQGPCCTAHCAFKSKTEKCRDDSDCAKEGICNGI 539
ADAM10_Maus KDDCCFDANQPEGKKCKLKPGKQCSPSQGPCCTAQCAFKSKSEKCRDDSDCAKEGICNGF 540
**:**:*******:********************:******:**********:******:

ADAM10_human TALCPASDPKPNFTDCNRHTQVCINGQCAGSICEKYGLEECTCASSDGKDDKELCHVCCM 599
ADAM10_bovine TALCPASDPKPNFTDCNRHTQVCINGQCAGSICEKHGLEECTCASSDGKDDKELCHVCCM 599
ADAM10_Maus TALCPASDPKPNFTDCNRHTQVCINGQCAGSICEKYDLEECTCASSDGKDNKELCHVCCM 600
***********************************:.*************:*********

ADAM10_human KKMDPSTCASTGSVQWSRHFSGRTITLQPGSPCNDFRGYCDVFMRCRLVDADGPLARLKK 659
ADAM10_bovine KKMEPSTCASTGSVQWNKYFLGRTITLQPGSPCNDFRGYCDVFMRCRLVDADGPLARLKK 659
ADAM10_Maus KKMAPSTCASTGSLQWSKQFSGRTITLQPGSPCNDFRGYCDVFMRCRLVDADGPLARLKK 660
*** *********:**.: * ***************************************

ADAM10_human AIFSPELYENIAEWIVAHWWAVLLMGIALIMLMAGFIKICSVHTPSSNPKLPPPKPLPGT 719
ADAM10_bovine AIFSPELYENIAEWIVAYWWAVLLMGIALIMLMAGFIKICSVHTPSSNPKLPPPKPLPGT 719
ADAM10_Maus AIFSPQLYENIAEWIVAHWWAVLLMGIALIMLMAGFIKICSVHTPSSNPKLPPPKPLPGT 720
*****:***********:******************************************

ADAM10_human LKRRRPPQPIQQPQRQRPRESYQMGHMRR 748
ADAM10_bovine LKRRRPPQPIQQPQRQRPRESYQMGHMRR 748
ADAM10_Maus LKRRRPPQPIQQPPRQRPRESYQMGHMRR 749
************* ***************

## Patentansprüche

1. Rekombinante Zelle, die ein Substratprotein exprimiert, das mindestens einen Sequenzbereich von 18 Aminosäuren des humanen Amyloidvorläuferproteins (APP, amyloid precursor protein) umfasst oder das mindestens einen Sequenzbereich von 18 Aminosäuren eines Proteins umfaßt, welches zum humanen APP eine Sequenzidentität von 86% über mindestens 770 Aminosäuren besitzt, wobei der Sequenzbereich die α-Sekretasespaltstelle sowie 6 Aminosäurereste aminoterminal und 12 Aminosäurereste carboxyterminal bezogen auf die α-Sekretasespaltstelle enthält, und
a) entweder eine rekombinante Nukleinsäure enthält, umfassend mindestens ein Gen für die Protease ADAM 10 aus Rind (Bos taurus) oder Mensch oder ein Gen für ein Proteaseprotein, das mit der Disintegrin-Metalloprotease ADAM 10 aus dem Rind (*Bos taurus*) oder aus dem Menschen eine Sequenzidentität von mindestens 94 % über 824 Aminosäuren auf Aminosäureebene aufweist, wobei das Gen oder die Gene unter der Kontrolle eines heterologen Promotors stehen,
b) oder heterologe RNA codierend für das besagte Proteaseprotein nach Alternative a) enthält.

2. Rekombinante Zelle, **dadurch gekennzeichnet, daß** sie rekombinante Nukleinsäure enthält, umfassend entweder
a) mindestens ein Gen für ein Substratprotein, das mindestens einen Sequenzbereich von 18 Aminosäuren des humanen Amyloidvorläuferproteins (APP, amyloid precursor protein) oder eines homologen Proteins umfasst oder das mindestens einen Sequenzbereich von 18 Aminosäuren eines Proteins umfaßt, welches zum humanen APP eine Sequenzidentität von 86% über mindestens 770 Aminosäuren besitzt, wobei der Sequenzbereich die α-Sekretasespaltstelle sowie 6 Aminosäurereste aminoterminal und 12 Aminosäurereste carboxyterminal bezogen auf die α-Sekretasespaltstelle enthält, und
b) mindestens ein Gen für die Protease ADAM 10 aus Rind (Bos taurus) oder Mensch oder ein Gen für ein Proteaseprotein, das mit der Disinteorin-Metalloprotease ADAM 10 aus dem Rind (*Bos taurus*) oder aus dem Menschen eine Sequenzidentität von mindestens 94 % über 824 Aminosäuren auf Aminosäureebene aufweist, wobei das Gen oder die Gene unter der Kontrolle eines heterologen Promotors stehen,
oder **dadurch gekennzeichnet, daß** die Zelle heterologe RNA kodierend für das besagte Substratprotein nach a) und das besagte Proteaseprotein nach b) enthält.

3. Rekombinante Zelle, **dadurch gekennzeichnet, daß** sie rekombinante Nukleinsäure(n) enthält, die codieren:
a) für eine dominant negative Form der Disintegrin-Metalloprotease ADAM 10 aus dem Rind (*Bos taurus*) oder aus dem Menschen, wobei es sich bei der dominant negativen Form der Disintegrin-Metalloprotease ADAM 10 um die Mutante E384A der Disintegrin-Metalloprotease ADAM 10 aus dem Rind oder um eine Mutante aus dem Menschen mit derselben dominant negativen Funktion handelt, und
b) gegebenenfalls für ein Substratprotein wie in Anspruch 2 definiert.

4. Zelle nach einem der Ansprüche 1 bis 3, **dadurch gekennzeichnet, daß** das Substratprotein ein Fusionsprotein ist, bei dem der aminoterminale Sequenzbereich des Fusionsproteins von einem Markerprotein, einem Enzym oder einem katalytisch wirksamen Teil eines Enzyms und der carboxyterminale Sequenzbereich des Fusionsproteins von dem humanen Amyloidvorläuferprotein oder einem Teil des humanen Amyloidvorläuferproteins gebildet werden.

5. Zelle nach Anspruch 4, **dadurch gekennzeichnet, daß** es sich bei dem Enzym um eine um mindestens 24 Aminosäurereste carboxyterminal verkürzte alkalische Phosphatase SEAP handelt.

6. Zelle nach Anspruch 5, **dadurch gekennzeichnet, daß** das Substratprotein eine Sequenz gemäß SEQ ID-No:1 aufweist.

7. Verfahren zur Auffindung pharmazeutischer Wirksubstanzen, **gekennzeichnet durch** die folgenden Schritte
a) Bereitstellung einer Zelle gemäß Anspruch 1,
b) Applikation einer Testsubstanz auf eine Zelle, auf eine Zellfraktion oder auf ein Gemisch von Zellfraktionen, die jeweils sowohl das Substratprotein als auch das Proteaseprotein umfassen, und
c) Quantitative Bestimmung eines seit der Applikation der Testsubstanz gebildeten Proteolyseproduktes des Substratproteins.

8. Verfahren zur Auffindung von weiteren Sekretasen oder von pharmazeutischen Wirksubstanzen, umfassend die folgenden Schritte
a) Expression eines Substratproteins und einer dominant negativen Form der Disintegrin-Metalloprotease ADAM 10 aus dem Rind (*Bos taurus*) oder aus dem Menschen in einer Zelle gemäß Anspruch 3,
b) Expression eines weiteren Testproteins in der Zelle und/oder Applikation einer Testsubstanz auf die Zelle oder auf eine Zellfraktion dieser Zelle, und
c) Quantitative Bestimmung eines gebildeten Proteolyseproduktes des Substratproteins.

9. Verfahren nach einem der Ansprüche 7 und 8, **dadurch gekennzeichnet, daß** die quantitative Bestimmung des Proteolyseproduktes durch einen enzymatischen Test erfolgt, bei dem von der katalytischen Aktivität einer Probe auf die Menge an einem bestimmten Proteolyseprodukt in der Probe geschlossen wird.

10. Verfahren nach einem der Ansprüche 7 und 8, **dadurch gekennzeichnet, daß** die quantitative Bestimmung des Proteolyseproduktes durch einen spektroskopischen Test erfolgt, bei dem von der spektroskopischen Eigenschaft einer Probe auf die Menge an einem bestimmten Proteolyseprodukt in der Probe geschlossen wird.

11. Verwendung eines Proteaseproteins wie in Anspruch 1 oder 2 definiert zur proteolytischen Spaltung eines Substratproteins wie in Anspruch 1 oder 2 definiert in vitro.

12. Verwendung einer dominant negativen Form der Disintegrin-Metalloprotease ADAM 10 aus dem Rind (*Bos taurus*) oder aus dem Menschen oder einer Nukleinsäure, codierend für eine dieser dominant negativen Formen, zur Unterdrückung der α-Sekretaseaktivität von zellulären Testsystemen in vitro.

13. Verfahren zur Bestimmung des Risikofaktors für neurodegenerative Erkrankungen, insbesondere für *Morbus Alzheimer,* **dadurch gekennzeichnet, daß**
a) eine DNA-Probe der Testperson unter Einsatz genspezifischender Primer gegen die humane Disintegrin-Metalloprotease ADAM 10 der PCR-Analyse unterworfen wird,
b) und die Sequenz des Amplifikationsproduktes mit der Sequenz der humanen Disintegrin-Metalloprotease ADAM 10 verglichen wird.

14. Verfahren zur Bestimmung des Risikofaktors für neurodegenerative Erkrankungen, insbesondere für *Morbus Alzheimer,* durch quantitative Bestimmung der mRNA-Mengen von humanem ADAM 10 aus Zell- und Gewebeproben, umfassend einen der folgenden Schritte:
a) Isolierung und Quantifizierung der ADAM 10-mRNA,
b) Umschreiben der ADAM 10-RNA in einzel- oder doppelsträngige cDNA, welche dann quantifiziert wird,
c) Einsetzen der gesamten Nukleotidsequenz von ADAM 10 oder ihrer Teile zur Quantifizierung mittels Hochdurchsatz-Expressionsanalyse.

15. Verfahren zur Bestimmung des Risikofaktors für neurodegenerative Erkrankungen, insbesondere für *Morbus Alzheimer,* **dadurch gekennzeichnet, daß** die tatsächliche chromosomalen Lokalisierung des Gens der Disintearin-Metalloprotease ADAM 10 mit seiner normalen chromosomalen Lokalisierung, Chromosom 15 bei 12.44 cR₃₀₀₀, verglichen wird.

16. Arzneimittel, enthaltend eine rekombinante Nukleinsäure, die für ein Proteaseprotein wie in Anspruch 1 oder 2 definiert codiert, zur Behandlung der Alzheimerschen Krankheit unter Erhöhung der alpha-Sekretase-Aktivität

17. Arzneimittel, enthaltend eine Nukleinsäure, die für die Disintegrin-Metalloprotease ADAM 10 aus Rind oder Mensch kodiert oder für ein Proteaseprotein kodiert, das mit der Disintegrin-Metalloprotease ADAM 10 aus dem Rind oder aus dem Menschen eine Sequenzidentität von mindestens 94 % über 824 Aminosäuren auf Aminosäureebene aufweist.

18. Arzneimittel, umfassend die nicht-membranverankerte lösliche Form eines Proteaseproteins wie in Anspruch 1 oder 2 definiert, insbesondere die lösliche Form von humanem ADAM 10 zur Behandlung der Alzheimerschen Krankheit unter Erhöhung der alpha-Sekretase-Aktivität.

19. Verwendung von Antikörpern oder Antikörperderivaten, die ein Proteaseprotein wie in Anspruch 1 oder 2 definiert spezifisch binden, zum quantitativen in vitro-Nachweis der alpha-Sekretase und/oder zur in vitro-Diagnose einer Prädisposition zur Ausbildung der Alzheimerschen Krankheit.

20. Verfahren zur Bestimmung des Aktivitätszustands von humanem ADAM 10 durch Quantifizierung des Phosphorylierungsgrades von humanem ADAM 10 in Zell- und Gewebeproben, wobei entweder
a) ADAM 10 isoliert wird und sein Phosphorylierungsgrad entweder durch Massenspektrometrie, durch spezifische Anti-Phosphothreonin-Antikörper, durch Quantifizierung der Aminosäure Phosphothreonin oder durch Dephosphorylierung (Phosphatase-Assay) von ADAM 10 bestimmt wird, oder
b) Zell- oder Gewebeproben direkt in eine Proteomanalyse eingesetzt werden und der Nachweis von phosphoryliertem ADAM 10 wie unter Punkt a) beschrieben erfolgt.

21. Transgenes, nicht menschliches Tier, das rekombinante Nukleinsäure, wie in Anspruch 1 oder 2 definiert, aufweist und diese exprimiert.

22. Verwendung eines transgenen, nicht menschlichen Tieres nach Anspruch 21 als invivo-Modell zur Identifikation und Charakterisierung von alpha-Sekretaseaktivierenden Substanzen.

23. Verwendung einer rekombinanten Nukleinsäure, codierend für ein Proteaseprotein wie in Anspruch 1 oder 2 definiert, zur Erhöhung der alpha-Sekretaseaktivität in vitro.

24. Transgenes, nicht menschliches Tier, das rekombinante Nukleinsäure, codierend für eine dominant negative Form der Disintegrin-Metalloprotease ADAM 10 aus dem Menschen oder dem Rind aufweist, wie in Anspruch 3 definiert, und diese exprimiert.

25. Transgenes nicht-menschliches Tier gemäß Anspruch 24, **dadurch gekennzeichnet, daß** es als *in vivo*-Modell eines Organismus mit einer Prädisposition zur Ausbildung der Alzheimerschen Krankheit zur Identifikation und Charakterisierung von neuen Arzneimittel-Wirkstoffen gegen die Alzheimersche Krankheit verwendet wird.

26. Transgenes nicht-menschliches Tier gemäß Anspruch 24, **dadurch gekennzeichnet, daß** es zur Identifikation und Charakterisierung neuer Proteasen mit alpha-Sekretaseaktivität verwendet wird.

27. Verwendung eines transgenen, nicht menschlichen Tieres, das rekombinante Nukleinsäure, codierend für eine dominant negative Form der Disintegrin-Metalloprotease ADAM 10 aus dem Menschen oder dem Rind wie in Anspruch 3 definiert, aufweist und diese exprimiert, zur Identifikation und Charakterisierung von neuen Arzneimittel-Wirkstoffen gegen die Alzheimerschen Krankheit.

28. Verwendung eines transgenen, nicht menschlichen Tieres, das rekombinante Nukleinsäure, codierend für eine dominant negative Form der Disintegrin-Metalloprotease ADAM 10 aus dem Menschen oder dem Rind aufweist und diese exprimiert, zur Identifikation und Charakterisierung neuer Proteasen mit alpha-Sekretaseaktivität.

## Claims

1. A recombinant cell which expresses a substrate protein which comprises at least one sequence region of 18 amino acids of the human amyloid precursor protein (APP, amyloid precursor protein) or which comprises at least one sequence region of 18 amino acids of a homologous protein which shows sequence identity of 86 % over at least 770 amino acids with human APP, the sequence region containing the α-secretase cleavage site and 6 amino acid residues amino-terminally and 12 amino acid residues carboxy-terminally relative to the α-secretase cleavage site, and
a) either contains recombinant nucleic acid, comprising at least one gene for the protease ADAM 10 from cattle (*Bos taurus*), or from humans, or from a gene for a protease protein, which shows sequence identity of at least 94 % over 824 amino acids at the amino acid level with the disintegrin metalloprotease ADAM 10, the gene or the genes being under the control of a heterologous promoter,
b) or contains heterologous RNA coding for the aforesaid protease protein according to alternative a)

2. A recombinant cell, **characterized in that** it contains a recombinant nucleic acid, comprising either
a) at least one gene for a substrate protein which comprises at least one sequence region of 18 amino acids of the human amyloid precursor protein (APP, amyloid precursor protein) or of a homologous protein, or which comprises at least one sequence region of 18 amino acids of a protein which shows sequence identity of 86 % over at least 770 amino acids with human APP, the sequence region containing the α-secretase cleavage site and 6 amino acid residues amino-terminally and 12 amino acid residues carboxy-terminally relative to the α-secretase cleavage site, and
b) at least one gene for the protease ADAM 10 from cattle (*Bos taurus),* or from humans, or a gene for a protease protein, which shows sequence identity on amino acid level of at least 94% over 824 amino acids with the disintegrin metalloprotease ADAM 10, the gene or the genes being under the control of a heterologous promoter,
or **characterized in that** the cell contains heterologous RNA coding for the aforesaid substrate protein according to a) and for the aforesaid protease protein according to b).

3. A recombinant cell, **characterized in that** it contains recombinant nucleic acid(s) which code(s):
a) for a dominantly negative form of the disintegrin metalloprotease ADAM 10 from cattle (*Bos taurus*) or from humans, the dominantly negative form of the disintegrin metalloprotease ADAM 10 being the mutant E384A of the disintegrin metalloprotease ADAM 10 from cattle or a mutant from humans with the same dominantly negative function, and
b) optionally for a substrate protein as set forth in claim 2.

4. The cell of any one of claims 1 to 3, **characterized in that** the substrate protein is a fusion protein in which the amino-terminal sequence region of the fusion protein is formed from a marker protein, an enzyme or a catalytically active part of an enzyme, and the carboxy-terminal sequence region of the fusion protein is formed from the human amyloid precursor protein or a part of the human amyloid precursor protein.

5. The cell of claim 4, **characterized in that** the enzyme is an alkaline phosphatase SEAP truncated carboxy-terminally by at least 24 amino acid residues.

6. The cell of claim 5, **characterized in that** the substrate protein has a sequence as in SEQ ID No.:1.

7. A method for the discovery of pharmaceutical active substances, **characterized by** the following steps
a) preparation of the cell according to claim 1,
b) administration of a test substance to a cell, to a cell fraction or to a mixture of cell fractions, which in each case comprises both the substrate protein and the protease protein, and
c) quantitative determination of a proteolysis product formed from the substrate protein since the administration of the test substance.

8. A method for the discovery of further secretases or of pharmaceutical active substances, comprising the following steps:
a) expression of a substrate protein and of a dominantly negative form of the disintegrin metalloprotease ADAM 10 from cattle (*Bos taurus*), from humans in the cell of claim 3,
b) expression of a further test protein in the cell and/or administration of a test substance to the cell or to a cell fraction of this cell, and
c) quantitative determination of a proteolysis product formed from the substrate protein.

9. The method of claim 7 and 8, **characterized in that** the quantitative determination of the proteolysis product is carried out by means of an enzymatic test in which a conclusion is made as to the amount of a certain proteolysis product in the sample from the catalytic activity of a sample.

10. The method of claim 7 and 8, **characterized in that** the quantitative determination of the proteolysis product is carried out by means of an enzymatic test in which a conclusion is made as to a certain proteolysis product in the sample from the catalytic activity of a sample.

11. Use of a protease protein as set forth in claim 1 or 2 for *in vitro* proteolytic cleavage of a substrate protein as set forth in claim 1 or 2.

12. Use of a dominantly negative form of the disintegrin metalloprotease ADAM 10 from cattle (*Bos taurus)* or from humans, or from a nucleic acid coding for one of these dominantly negative forms to suppress the α-secretase activity of cellular test systems.

13. A method for the determination of a risk factor for neurodegenerative diseases, in particular for *Alzheimer's disease,* **characterized in that**
a) a DNA sample of the test person is subjected to PCR analysis using gene-specific primers against the human disintegrin metalloprotease ADAM 10,
b) and the sequence of the amplification product is compared with the known sequence of the human disintegrin metalloprotease ADAM 10.

14. A method for the determination of a risk factor for neurodegenerative diseases, in particular for *Alzheimer's disease,* by quantitative determination of the amounts of mRNA of human ADAM 10 from cell and tissue samples, comprising one of the following steps:
a) isolation and quantification of the ADAM 10 mRNA,
b) the transcription of ADAM 10-RNA in single- or double-stranded cDNA which is then quantified,
c) the entire nucleotide sequence of ADAM 10 or parts thereof is employed for the quantification by means of high-throughput expression analysis

15. A method for the determination of a risk factor for neurodegenerative diseases, in particular for *Alzheimer's disease,* **characterized in that** the actual chromosomal localization of the gene of the disintegrin metalloprotease ADAM 10 is compared with its normal chromosomal location, chromosome 15 at 12.44 cR₃₀₀₀.

16. A medicament comprising a recombinant nucleic acid which codes for a protease protein as set forth in claim 1 and 2 for treatment of *Alzheimer's disease* with increasing the α-secretase activity.

17. A medicament comprising a nucleic acid which codes for the disintegrin metalloprotease ADAM 10 from cattle or humans or for a protease protein which shows on amino acid level at least 94 % sequence identity over 824 amino acids with the disintegrin metalloprotease from cattle or from humans.

18. A medicament comprising the nonmembrane-anchored soluble form of a protease protein as set forth in claims 1 or 2, in particular the soluble form of human ADAM 10 for treatment of *Alzheimer's disease* with increasing the α-secretase activity.

19. Use of antibodies or antibody derivates which specifically bind a protease protein as set forth in claims 1 or 2 for quantitative detection of α-secretase *in vitro* and/or for diagnosis of a predisposition to the development *of Alzheimer's disease.*

20. A method for the determination of the activity state of human ADAM 10 by quantification of the degree of phosphorylation of human ADAM 10 in cell and tissue samples, in which either
a) ADAM 10 is isolated and its degree of phosphorylation is determined either by mass spectrometry, by means of specific anti-phosphothreonine antibodies, by quantification of the amino acid phosphothreonine or by dephosphorylation (phosphatase assay) of ADAM 10, or
b) cell or tissue samples are employed directly in a proteome analysis and the detection of a phosphorylated ADAM 10 is carried out as described under item a).

21. A transgenic non human animal which contains and expresses recombinant nucleic acid as set forth in claim 1 or 2.

22. Use of the transgenic non human animal of claim 21 as an *in vivo* model for the identification and characterization of α-secretase activating substances.

23. Use of the recombinant nucleic acid, coding for a protease protein as set forth in claim 1 or 2, for increasing the α-secretase activity *in vitro.*

24. A transgenic non human animal which contains recombinant nucleic acid coding for a dominantly negative form of the disintegrin metalloprotease ADAM 10 from humans or from cattle as set forth in claim 3, and which expresses this nucleic acid.

25. The transgenic non human animal as set forth in claim 24, **characterized in that** it is used as an *in vivo* model of an organism with a predisposition to the development of *Alzheimer's disease* for the identification and characterization of new active agents against *Alzheimer's disease.*

26. The transgenic non human animal as set forth in claim 24, **characterized in that** it is used for the identification and characterization of new proteases with α-secretase activity.

27. Use of a transgenic non human animal which contains recombinant nucleic acid, coding for a dominantly negative form of the disintegrin metalloprotease ADAM 10 from humans or cattle as set forth in claim 3, and which expresses this nucleic acid, for the identification and characterization of new active agents against *Alzheimer's disease.*

28. Use of a transgenic non human animal which contains recombinant nucleic acid, coding for a dominantly negative form of the disintegrin metalloprotease ADAM 10 from humans or cattle, and which expresses this nucleic acid, for the identification and characterization of new proteases with α-secretase activity.

## Revendications

1. Cellule recombinante qui exprime une protéine substrat qui comprend au moins un segment de séquence de 18 aminoacides de la protéine précurseur de l'amyloïde (APP, *amyloid precursor protein*) humaine ou qui comprend au moins un segment de séquence de 18 aminoacides d'une protéine ayant une identité de séquence de 86 % sur au moins 770 aminoacides avec l'APP humaine, le segment de séquence contenant le site de coupure de l'α-secrétase ainsi que 6 résidus aminoacides à l'extrémité amino-terminale et 12 résidus aminoacides à l'extrémité carboxy-terminale par rapport au site de coupure del'α-secrétase, et
a) soit contient un acide nucléique recombinant comprenant au moins un gène codant pour la protéase ADAM 10 bovine (de *Bos taurus*) ou humaine, ou un gène codant pour une protéine protéase qui présente sur le plan des aminoacides une identité de séquence d'au moins 94 % sur 824 aminoacides avec la désintégrine-métalloprotéase ADAM 10 bovine (de *Bos taurus)* ou humaine, le gène ou les gènes étant sous le contrôle d'un promoteur hétérologue,
b) soit contient de l'ARN hétérologue codant pour ladite protéine protéase selon la possibilité a).

2. Cellule recombinante, **caractérisée en ce qu'**elle contient un acide nucléique recombinant qui comprend soit
a) au moins un gène codant pour une protéine substrat qui comprend au moins un segment de séquence de 18 aminoacides de la protéine précurseur de l'amyloïde (APP, *amyloid precursor protein*) humaine ou d'une protéine homologue ou qui comprend au moins un segment de séquence de 18 aminoacides d'une protéine ayant une identité de séquence de 86 % sur au moins 770 aminoacides avec l'APP humaine, le segment de séquence contenant le site de coupure de l'α-secrétase ainsi que 6 résidus aminoacides à l'extrémité amino-terminale et 12 résidus aminoacides à l'extrémité carboxy-terminale par rapport au site de coupure de l'α-secrétase, et
b) au moins un gène codant pour la protéase ADAM 10 bovine (de *Bos taurus*)
ou humaine, ou un gène codant pour une protéine protéase qui présente sur le plan des aminoacides une identité de séquence d'au moins 94 % sur 824 aminoacides avec la désintégrine-métalloprotéase ADAM 10 bovine (de *Bos taurus)* ou humaine, le gène ou les gènes étant sous le contrôle d'un promoteur hétérologue,
ou **caractérisée en ce que** la cellule contient de l'ARN hétérologue codant pour
ladite protéine substrat selon a) et ladite protéine protéase selon b).

3. Cellule recombinante, **caractérisée en ce qu'**elle contient un(des) acide(s) nucléique(s) recombinant(s) qui code(nt) :
a) pour une forme dominante négative de la désintégrine-métalloprotéase ADAM 10 bovine (de *Bos taurus*) ou humaine, la forme dominante négative de la désintégrine-métalloprotéase ADAM 10 consistant en le mutant E384A de la désintégrine-métalloprotéase ADAM 10 d'origine bovine ou en un mutant d'origine humaine ayant la même fonction dominante négative, et
b) éventuellement pour une protéine substrat telle que définie dans la revendication 2.

4. Cellule selon l'une quelconque des revendications 1 à 3, **caractérisée en ce que** la protéine substrat est une protéine de fusion dans laquelle sont insérés le segment de séquence amino-terminal de la protéine de fusion d'une protéine de marquage, d'une enzyme ou d'une partie à activité catalytique d'une enzyme et le segment de séquence carboxy-terminal de la protéine de fusion de la protéine précurseur de l'amyloïde humaine ou d'une partie de la protéine précurseur de l'amyloïde humaine.

5. Cellule selon la revendication 4, **caractérisée en ce que** l'enzyme consiste en une phosphatase alcaline SEAP raccourcie d'au moins 24 aminoacides à l'extrémité carboxy-terminale.

6. Cellule selon la revendication 5, **caractérisée en ce que** la protéine substrat présente une séquence selon SEQ ID n° 1.

7. Procédé pour trouver des substances actives pharmaceutiques, **caractérisé par** les étapes suivantes
a) fourniture d'une cellule selon la revendication 1,
b) application d'une substance d'essai sur une cellule, sur une fraction cellulaire ou sur un mélange de fractions cellulaires, qui comprennent chacune aussi bien la protéine substrat que la protéine protéase, et
c) détermination quantitative d'un produit de protéolyse de la protéine substrat, formé depuis l'application de la substance d'essai.

8. Procédé pour trouver de nouvelles secrétases ou des substances actives pharmaceutiques, **caractérisé par** les étapes suivantes
a) expression d'une protéine substrat et d'une forme dominante négative de la désintégrine-métalloprotéase ADAM 10 bovine (de *Bos taurus*) ou humaine dans une cellule selon la revendication 3,
b) expression d'une autre protéine d'essai dans la cellule et/ou application d'une substance d'essai sur la cellule ou sur une fraction de cette cellule, et
c) détermination quantitative d'un produit de protéolyse formé de la protéine substrat.

9. Procédé selon l'une quelconque des revendications 7 et 8, **caractérisé en ce que** la détermination quantitative du produit de protéolyse s'effectue par un essai enzymatique dans lequel on déduit de l'activité catalytique d'un échantillon la quantité d'un produit de protéolyse déterminé dans l'échantillon.

10. Procédé selon l'une quelconque des revendications 7 et 8, **caractérisé en ce que** la détermination quantitative du produit de protéolyse s'effectue par un essai spectroscopique dans lequel on déduit de la propriété spectroscopique d'un échantillon la quantité d'un produit de protéolyse déterminé dans l'échantillon.

11. Utilisation d'une protéine protéase telle que définie dans la revendication 1 ou 2, pour la coupure protéolytique in vitro d'une protéine substrat telle que définie dans la revendication 1 ou 2.

12. Utilisation d'une forme dominante négative de la désintégrine-métalloprotéase ADAM 10 bovine (de *Bos taurus)* ou humaine ou d'un acide nucléique codant pour une de ces formes dominantes négatives, pour la répression de l'activité α-secrétase de systèmes d'essai cellulaires in vitro.

13. Procédé pour la détermination du facteur de risque de maladies neurodégénératives, en particulier de la maladie d'Alzheimer, **caractérisé en ce que**
a) on soumet à l'analyse par PCR un échantillon d'ADN de l'individu à tester, en utilisant des amorces spécifiques de gènes contre la désintégrine-métalloprotéase ADAM 10 humaine,
b) et on compare la séquence du produit d'amplification à la séquence de la désintégrine-métalloprotéase ADAM 10 humaine.

14. Procédé pour la détermination du facteur de risque de maladies neurodégénératives, en particulier de la maladie d'Alzheimer, par détermination quantitative des quantités d'ARNm d'ADAM 10 humaine provenant d'échantillons de cellules et de tissus, comprenant l'une des étapes suivantes :
a) isolement et détermination quantitative de l'ARNm d'ADAM 10,
b) transcription de l'ARN d'ADAM 10 en ADNc simple brin ou double brin, qui est ensuite quantifié,
c) utilisation de toute la séquence nucléotidique d'ADAM 10 ou de parties de celle-ci pour la détermination quantitative par analyse d'expression à haut débit.

15. Procédé pour la détermination du facteur de risque de maladies neurodégénératives, en particulier de la maladie d'Alzheimer, **caractérisé en ce qu'**on compare la localisation chromosomique réelle du gène de la désintégrine-métalloprotéase ADAM 10 à sa localisation chromosomique normale, chromosome 15 à 12.44 cR₃₀₀₀.

16. Médicament contenant un acide nucléique recombinant qui code pour une protéine protéase telle que définie dans la revendication 1 ou 2, destiné au traitement de la maladie d'Alzheimer avec augmentation de l'activité alpha-secrétase.

17. Médicament contenant un acide nucléique qui code pour la désintégrine-métalloprotéase ADAM 10 bovine ou humaine ou pour une protéine protéase qui présente sur le plan des aminoacides une identité de séquence d'au moins 94 % sur 824 aminoacides avec la désintégrine-métalloprotéase ADAM 10 bovine ou humaine.

18. Médicament comprenant la forme soluble, non ancrée à la membrane, d'une protéine protéase telle que définie dans la revendication 1 ou 2, en particulier la forme soluble d'ADAM 10 humaine, pour le traitement de la maladie d'Alzheimer avec augmentation de l'activité α-secrétase.

19. Utilisation d'anticorps ou de dérivés d'anticorps, qui se lient spécifiquement à une protéine protéase telle que définie dans la revendication 1 ou 2, pour la détermination quantitative in vitro de l'alpha-secrétase et/ou pour le diagnostic in vitro d'une prédisposition à l'apparition de la maladie d'Alzheimer.

20. Procédé pour la détermination de l'état d'activité d'ADAM 10 humaine par détermination quantitative du degré de phosphorylation d'ADAM 10 humaine dans des échantillons de cellules et de tissus, dans lequel soit
a) on isole ADAM 10 et on détermine son degré de phosphorylation soit par spectrométrie de masse, soit au moyen d'anticorps anti-phosphothréonine spécifiques, ou par détermination quantitative de l'aminoacide phosphothréonine ou par déphosphorylation (essai à la phosphatase) d'ADAM 10, soit
b) on utilise directement des échantillons de cellules ou de tissus dans une analyse du protéome et on effectue la détection d'ADAM 10 phosphorylée, comme décrit au point a).

21. Animal non humain transgénique qui comporte de l'acide nucléique recombinant tel que défini dans la revendication 1 ou 2 et l'exprime.

22. Utilisation d'un animal non humain transgénique selon la revendication 21, en tant que modèle in vivo pour l'identification et la caractérisation de substances activant l'alpha-secrétase.

23. Utilisation d'un acide nucléique recombinant, codant pour une protéine protéase telle que définie dans la revendication 1 ou 2, pour l'augmentation de l'activité alpha-secrétase in vitro.

24. Animal non humain transgénique qui comporte de l'acide nucléique recombinant qui code pour une forme dominante négative de la désintégrine-métalloprotéase humaine ou bovine, telle que définie dans la revendication 3, et l'exprime.

25. Animal non humain transgénique selon la revendication 24,
**caractérisé en ce qu'**il est utilisé en tant que modèle in vivo d'un organisme ayant une prédisposition à l'apparition de la maladie d'Alzheimer, pour l'identification et la caractérisation de nouveaux principes actifs-médicaments contre la maladie d'Alzheimer.

26. Animal non humain transgénique selon la revendication 24,
**caractérisé en ce qu'**il est utilisé pour l'identification et la caractérisation de nouvelles protéases à activité alpha-secrétase.

27. Utilisation d'un animal non humain transgénique qui comporte de l'acide nucléique recombinant qui code pour une forme dominante négative de la désintégrine-métalloprotéase ADAM 10 humaine ou bovine, telle que définie dans la revendication 3, et l'exprime, pour l'identification et la caractérisation de nouveaux principes actifs-médicaments contre la maladie d'Alzheimer.

28. Utilisation d'un animal non humain transgénique qui comporte de l'acide nucléique recombinant qui code pour une forme dominante négative de la désintégrine-métalloprotéase ADAM 10 humaine ou bovine, et l'exprime, pour l'identification et la caractérisation de nouvelles protéases à activité alpha-secrétase.
